# EUROPEAN PATENT APPLICATION

(11) **EP 4 711 444 A2**
(43) Date of publication of application: **18.03.2026**
(21) Application number: 25213796.3
(22) Date of filing: 25.06.2021
(51) Int. Cl.: C12N 5/00

(54) **ENDOTHELIAL CELLS FOR MITIGATION OF CHEMOTHERAPY-INDUCED TOXICITY**

(30) Priority: 25.06.2020 US 202063044243 P; 12.05.2021 US 202163187486 P
(62) Divisional of application: 21829250.6
(71) Applicant: Angiocrine Bioscience, Inc., San Diego, CA 92121 (US)
(72) Inventor: FINNEGAN, Paul William, San Diego, CA 92121 (US); FRASER, John, K., San Diego, CA 92121 (US); GINSBERG, Michael Daniel, San Diego, CA 92121 (US)
(74) Representative: Barker Brettell LLP

(57) **Abstract**

The present invention provides compositions and methods for the mitigation of side effects of chemotherapy, for example in human subjects with hematologic malignancies (such as lymphoma, leukemia and myelodysplastic syndrome) as well as subjects with other malignancies or other conditions that may be treated with chemotherapy, such as high dose therapy (HDT) or a combination of high dose HDT and a hematopoietic stem cell transplant. The methods comprise administration of endothelial cells, such as engineered human umbilical vein endothelial cells engineered to express the adenoviral E4ORF1 protein (E4ORF1+ HUVECs), to human subjects. The side effects mitigated by the compositions and methods of the invention include, but are not limited to, oral / gastrointestinal side effects and febrile neutropenia.

## Description

### CROSS-REFERENCE TO RELATED APPLICATIONS

This application claims the benefit of priority of U.S. Provisional Patent Application No. 63/044,243 filed on June 25, 2020 and U.S. Provisional Patent Application No. 63/187,486 filed on May 12, 2021, the contents of each of which are hereby incorporated by reference in their entireties.

### SEQUENCE LISTING

The instant application contains a Sequence Listing which has been submitted electronically in ASCII format and is hereby incorporated by reference in its entirety. Said ASCII copy, created on June 25, 2021, is named Angiocrine_029_WO1_SL.txt and is 1,543 bytes in size.

### INCORPORATION BY REFERENCE

For the purpose of only those jurisdictions that permit incorporation by reference, all of the references cited in this disclosure are hereby incorporated by reference in their entireties. In addition, any manufacturers' instructions or catalogues for any products cited or mentioned herein are incorporated by reference. Documents incorporated by reference into this text, or any teachings therein, can be used in the practice of the present invention.

### BACKGROUND

It is well known that the use of chemotherapy is associated with side effects. Indeed, chemotherapeutic agents used in the treatment of life-threatening diseases such as cancer are often associated with a high risk of serious side effects such that, on many occasions, the dose of the chemotherapeutic agent that the patient receives is limited by the morbidity and mortality associated with the side-effects. For example, chemotherapy regimens used to treat malignancy or delivered as part of some treatments for autoimmune disease are often associated with dose-dependent toxicities including nausea, vomiting, diarrhea, infection, and gastrointestinal inflammation (mucositis). As these side-effects are related to the dose of chemotherapy delivered, they are particularly evident in patients in patients who have aggressive or relapsed disease who are deemed eligible to undergo high dose chemotherapy, for example high dose therapy (HDT) in association with hematopoietic stem cell transplantation. In such cases the patient frequently suffers from a syndrome of severe regimen related toxicities (SRRTs) involving multiple organ systems, including the oral-gastrointestinal (GI), pulmonary, central nervous system (CNS), hepatic, renal, and cardiac systems. The majority of the toxicities caused by high dose therapy (HDT) occur acutely or sub-acutely. The majority resolve within 100 days. While severe toxicities rarely persist over 1 year (Pamukcuoglu et al., "Hematopoietic Cell Transplant-Related Toxicities and Mortality in Frail Recipients, " Biol. Blood Marrow Transplant, 2019; 25(12): 2454-2460), ongoing adverse effects can be evident for several years. Severity of such toxicities increases with concomitant administration of radiotherapy. There is also a higher incidence and severity of such toxicities in frailer and older patients.

The incidence of severe (Grade ≥3) non-hematologic toxicity syndrome has been reported to occur in 57-100% of patients receiving HDT +/- radiation therapy followed by hematopoietic stem cell transplant. The most common non-hematologic toxicities include febrile neutropenia (with a reported incidence in certain settings of 83-95%) and a "cluster" of oro-gastrointestinal toxicities including severe oral mucositis and severe 'breakthrough' or 'refractory' nausea, vomiting and diarrhea (referred to collectively as N/V/D). The reported incidence of Grade ≥3 stomatitis or mucositis ranges from 30-81%, while severe N/V/D has been reported to occur at a rate of 12-30%. Rarer but life-threatening non-hematologic toxicities include severe infections (13-44%), nervous system disorders (4-47%), pneumonitis or pneumonia (10-38%), hepatic disorders (2-19%), renal failure (1-9%), and cardiovascular disorders (3-26%) (Scordo et al, 2017, "A Comprehensive Assessment of Toxicities in Patients with Central Nervous System Lymphoma Undergoing Autologous Stem Cell Transplantation Using Thiotepa, Busulfan, and Cyclophosphamide Conditioning" Biol. Blood Marrow Transplant 23 (2017) 38-43), Perales et al., Real-World Economic Burden Associated with Transplantation-Related Complications, Biol Blood Marrow Transplant, 23 (2017) 1788-1794*,* Olivieri et al., A Comparison of the Conditioning Regimens BEAM and FEAM for Autologous Hematopoietic Stem Cell Transplantation in Lymphoma: An Observational Study on 1038 Patients From Fondazione Italiana Linfomi. Biol Blood Marrow Transplant, 2018. 24(9): p. 1814-182.

There is a pressing need in the art for new and improved therapies the treatment and prevention of severe toxicities related to the use of chemotherapeutic agents, including high dose therapy (HDT). The present invention addresses this need.

### SUMMARY OF THE INVENTION

The present invention is based, in part, on certain discoveries made in the course of performing Phase 1 clinical trials to assess the safety of administering engineered endothelial cells to human subjects with various hematologic malignancies undergoing high dose chemo therapy and hematopoietic stem cell transplantation. In performing these clinical studies, it was found that, not only were there no apparent negative side effects or safety issues associated with the endothelial cell administration, but also that administration of the endothelial cells mitigated numerous non-hematologic and hematologic severe regimen related toxicities (SSRTs) in the treated patients. It was also found that different doses of endothelial cells resulted in different effects - with some side effects being mitigated at both low and high doses and other side effects being preferentially mitigated at higher doses. For example, it was found that the occurrence of severe oro-gastrointestinal toxicities was eliminated and the occurrence of febrile neutropenia was reduced by around 75% following administration of endothelial cells to patients having lymphoma without CNS involvement. These and other discoveries are described in more detail in the Examples section of this patent disclosure.

Building on these discoveries, the present invention provides various new methods and compositions for the mitigation of side effects of chemotherapy, including high dose therapy (HDT), including, but not limited to, HDT administered to patients in conjunction with hematopoietic stem cell transplantation.

Accordingly, the present invention provides methods for mitigating the side effects of chemotherapy in subjects in need thereof, such methods comprising administering an effective amount of a therapeutic composition comprising endothelial cells to subjects that have been treated with chemotherapy, thereby mitigating one or more of the side effects of the chemotherapy in the subject. The present invention also provides therapeutic compositions comprising endothelial cells, for example for use in such methods. The present invention also provides endothelial cells for use in the preparation of a therapeutic composition (or medicament) for example use in such methods.

In some embodiments the endothelial cells are umbilical vein endothelial cells (UVECs). In some embodiments the endothelial cells are human umbilical vein endothelial cells (HUVECs). In some embodiments the endothelial cells are engineered to express a recombinant protein. In some embodiments the endothelial cells are engineered to express the adenovirus E4ORF1 protein - i.e., they are E4ORF1+ endothelial cells. In some embodiments the endothelial cells are E4PRF1+ HUVECS.

In some embodiments the subjects to whom the endothelial cells are administered are adults. In some embodiments the subjects to whom the endothelial cells are administered are ≥ 18 years old. In some embodiments the subjects to whom the endothelial cells are administered are ≥ 20 years old. In some embodiments the subjects to whom the endothelial cells are administered are ≥ 30 years old. In some embodiments the subjects to whom the endothelial cells are administered are ≥ 40 years old. In some embodiments the subjects to whom the endothelial cells are administered are ≥ 50 years old. In some embodiments the subjects to whom the endothelial cells are administered are ≥ 60 years old.

In some embodiments the subjects to whom the endothelial cells are administered have a disease or disorder that can be treated using a chemotherapeutic regimen that results in severe regimen-related toxicity (SRRT). In some embodiments the subjects to whom the endothelial cells are administered have a disease or disorder that can be treated using high dose therapy (HDT). In some embodiments the subjects to whom the endothelial cells are administered have a disease or disorder that can be treated using a combination of chemotherapy (e.g., high dose therapy (HDT)) and a hematopoietic stem cell transplant.

In some embodiments the subjects to whom the endothelial cells are administered have cancer (i.e., a malignancy). In some embodiments the subjects to whom the endothelial cells are administered have a hematologic cancer / malignancy. In some embodiments the subjects to whom the endothelial cells are administered have a non-hematologic cancer / malignancy.

In some embodiments the subjects to whom the endothelial cells are administered have lymphoma. In some embodiments the subjects to whom the endothelial cells are administered have Hodgkins's lymphoma (HL). In some embodiments the subjects to whom the endothelial cells are administered have non-Hodgkins's lymphoma (NHL). In some embodiments the subjects to whom the endothelial cells are administered have diffuse large B cell lymphoma (DLBCL). In some embodiments the subjects to whom the endothelial cells are administered have a T cell lymphoma (TCL). In some embodiments the subjects to whom the endothelial cells are administered have a lymphoma without CNS involvement. In some embodiments the subjects to whom the endothelial cells are administered have a lymphoma with CNS involvement (i.e., "CNS lymphoma"). In some embodiments the subjects to whom the endothelial cells are administered have relapsed lymphoma. In some embodiments the subjects to whom the endothelial cells are administered have refractory lymphoma - i.e., lymphoma that did not respond to, or responded poorly to, a prior treatment.

In some embodiments the subjects to whom the endothelial cells are administered have multiple myeloma.

In some embodiments the subjects to whom the endothelial cells are administered have leukemia. In some embodiments the subjects to whom the endothelial cells are administered have acute myeloid leukemia (AML). In some embodiments the subjects to whom the endothelial cells are administered have acute lymphoblastic leukemia (ALL).

In some embodiments the subjects to whom the endothelial cells are administered have myelodysplastic syndrome.

In some embodiments the subjects to whom the endothelial cells are administered have a non-hematologic cancer / malignancy selected from the group consisting of an adrenocortical tumor, alveolar soft part sarcoma, astrocytoma, breast cancer, bladder cancer, brain cancer, carcinoma, cervical cancer, chondrosarcoma, colorectal cancer, desmoid tumors, desmoplastic small round cell cancer, endocrine cancer, endodermal sinus tumor, endometrial cancer, epithelioid hemangioendothelioma, esophageal cancer, Ewing sarcoma, gastric cancer, germ cell tumor, glioma, heart cancer, hepatoblastoma, hepatocellular carcinoma, lip and/or oral cancer, lung cancer, melanoma, mesothelioma, nephroma, neuroblastoma, non-rhabdomyosarcoma soft tissue sarcoma, oropharangeal cancer, osteosarcoma, ovarian cancer, pancreatic cancer, paraspinal sarcoma, prostate cancer, rectal cancer, renal cell carcinoma, retinoblastoma, rhabdomyosarcoma, small cell lung cancer, synovial sarcoma, testicular cancer, throat cancer, thyroid cancer, urethral cancer, uterine sarcoma, and Wilms Tumor.

In some embodiments the subjects to whom the endothelial cells are administered have an autoimmune disease, such as such as rheumatoid arthritis, systemic lupus erythematosus (SLE), systemic sclerosis (diffuse or limited), multiple sclerosis, Crohn's Disease, type I diabetes, or juvenile idiopathic arthritis.

In some embodiments the chemotherapy is a chemotherapy that results in severe regimen-related toxicity (SRRT). In some embodiments the chemotherapy is high dose therapy (HDT). In some embodiments the chemotherapy is administered as part of a conditioning regimen to prepare the subject for receipt of a transplant - e.g., a hematopoietic stem cell transplant. In some embodiments conditioning regimen is a myeloablative conditioning regimen. In some embodiments conditioning regimen is reduced intensity conditioning.

In some embodiments the chemotherapy is a CNS-penetrant chemotherapy. For example, in some embodiments the subject has CNS lymphoma and the chemotherapy is a CNS-penetrant chemotherapy.

In some embodiments the chemotherapy is a combination chemotherapy comprising the administration of carmustine, etoposide, cytarabine, and melphalan ("BEAM"). In some embodiments the chemotherapy is combination chemotherapy comprising the administration of bendamustine, etoposide, cytarabine, and melphalan ("BeEAM"). In some embodiments the chemotherapy is a combination chemotherapy comprising the administration of fotemustine, etoposide, cytarabine, and melphalan ("FEAM"). In some embodiments the chemotherapy is a combination chemotherapy comprising the administration of etoposide, cytarabine, and melphalan ("EAM"). In some embodiments the chemotherapy is combination chemotherapy comprising the administration of cyclophosphamide, carmustine, and etoposide ("CBV"). In some embodiments the chemotherapy is combination chemotherapy comprising the administration of thiotepa, busulfan, and cyclophosphamide ("TBC") - which is a CNS-penetrant chemotherapeutic combination. In some embodiments the chemotherapy is combination chemotherapy comprising the administration of rituximab, dexamethasone, high-dose cytarabine, and cisplatin ("DHAP"). In some embodiments the chemotherapy is combination chemotherapy comprising the administration of busulfan, cyclophosphamide and fludarabine ("Bu/Cy/Flu").

In some embodiments the chemotherapy comprises the administration of or melphalan - with or without busulfan. In some embodiments the chemotherapy is or comprises melphalan. For example, in some embodiments the chemotherapy comprises treating the subjects with melphalan at a dose of about 140mg/m2. Similarly, in some embodiments the chemotherapy comprises treating the subjects with melphalan at a dose of about 200mg/m2.

In some embodiments the endothelial cells (or compositions comprising endothelial cells) are first administered to the subjects from 0 to 28 days after the subject first exhibits side effects from the chemotherapy. In some embodiments the endothelial cells (or compositions comprising endothelial cells) are first administered to the subjects from 0 to 7 days after the subject first exhibits side effects from the chemotherapy. In some embodiments the endothelial cells (or compositions comprising endothelial cells) are first administered to the subjects from 0 to 2 days after the subject first exhibits side effects from the chemotherapy. In some embodiments the endothelial cells (or compositions comprising endothelial cells) are first administered to the subjects from 0 to 1 days after the subject first exhibits side effects from the chemotherapy. In some embodiments the endothelial cells (or compositions comprising endothelial cells) are first administered to the subjects before the subject first exhibits side effects from the chemotherapy.

In some embodiments the endothelial cells (or compositions comprising endothelial cells) are first administered to the subjects from 0 to 28 days after the subject completes treatment with the chemotherapy. In some embodiments the endothelial cells (or compositions comprising endothelial cells) are first administered to the subjects from 0 to 7 days after the subject completes treatment with the chemotherapy. In some embodiments the endothelial cells (or compositions comprising endothelial cells) are first administered to the subjects from 0 to 2 days after the subject completes treatment with the chemotherapy. In some embodiments the endothelial cells (or compositions comprising endothelial cells) are first administered to the subjects from 0 to 1 days after the subject completes treatment with the chemotherapy.

In some embodiments the subjects to whom the endothelial cells are administered also receive a hematopoietic stem cell transplant. In some such embodiments the subject receives a hematopoietic stem cell transplant at the same time that the endothelial cells are administered to the subject. In some such embodiments the hematopoietic stem cell transplant and the endothelial cells are administered to the subject together - for example in the same intravenous (IV) infusion. In some such embodiments the endothelial cells are administered to the subject after the subject receives a hematopoietic cell transplant. In some such embodiments the endothelial cells are administered to the subject on the same day that subject receives a hematopoietic cell transplant i.e., on Day 0, where Day 0 is the day of the hematopoietic cell transplant). In some such embodiments the endothelial cells are administered to the subject one day after the subject receives a hematopoietic cell transplant (i.e., on Day 1). In some such embodiments the endothelial cells are administered to the subject two days after the subject receives a hematopoietic cell transplant (i.e., on Day 2). In some such embodiments the endothelial cells are administered to the subject three days after the subject receives a hematopoietic cell transplant (i.e., on Day 3). In some such embodiments the endothelial cells are administered to the subject about 2 hours after that subject receives a hematopoietic cell transplant. In some such embodiments the endothelial cells are administered to the subject about 3 hours after the subject receives a hematopoietic cell transplant. In some such embodiments the endothelial cells are administered to the subject about 4 hours after the subject receives a hematopoietic cell transplant. In some embodiments the hematopoietic stem cell transplant is an autologous hematopoietic stem cell transplant. In some embodiments the hematopoietic stem cell transplant is an allogenic hematopoietic stem cell transplant. For example, in some embodiments the subjects receive an allogeneic umbilical cord blood transplant (UCBT).

In some embodiments the subjects receive high-dose chemotherapy (i.e., high dose therapy) and an autologous hematopoietic stem cell transplant prior to administration of the endothelial cells. In some embodiments the subjects receive high-dose chemotherapy (i.e., high dose therapy) and an allogeneic hematopoietic stem cell transplant prior to administration of the endothelial cells.

In some embodiments the endothelial cells are administered to the subjects in a therapeutic composition that comprises endothelial cells at a concentration of about 5 million cells per ml. In some embodiments the endothelial cells are administered to the subjects in a therapeutic composition that comprises human serum albumin (HSA). In some embodiments the endothelial cells are administered to the subjects in a therapeutic composition that comprises Dextran40. In some embodiments the endothelial cells are administered to the subjects in a therapeutic composition that comprises DMSO.

In some embodiments the endothelial cells (e.g., compositions comprising endothelial cells) are administered to the subjects once - i.e., using a single administration/dosing regimen (e.g., on Day 0). In some embodiments endothelial cells (e.g., compositions comprising endothelial cells) are administered to the subjects twice - i.e., using a double administration/dosing regimen (e.g., first on Day 0, and then again on a subsequent day, for example on Day 1, 2, 3, 4, 5, 6 or 7). In some embodiments the endothelial cells (e.g., compositions comprising endothelial cells) are administered to the subjects on multiple occasions. For example, in one embodiment the endothelial cells (e.g., compositions comprising endothelial cells) are administered to the subject first on day 0, and then again on day 2, and then again on a day 4, and so on.

In some embodiments Day 0 (i.e., the day on which the subject receives the stem cell transplant) is from 0 to 28 days after the subject has received the chemotherapy (e.g., HDT). In some embodiments Day 0 is from 0 to 7 days after the subject has received the chemotherapy (e.g., HDT). In some embodiments Day 0 is from 0 to 2 days after the subject has received the chemotherapy (e.g., HDT). In some embodiments Day 0 is from 0 to 2 days after the subject has received the chemotherapy (e.g., HDT). Typically, the treating physician will determine the appropriate timing between the subject receiving the chemotherapy (e.g., HDT) and receiving the stem cell transplant, for example based on factors such as the specific chemotherapy used, institutional guidelines, etc.

In some embodiments the endothelial cells (or compositions comprising endothelial cells) are administered to the subjects intravenously, for example by (IV) infusion. In some embodiments the IV infusion is administered peripherally, e.g., using a peripheral IV catheter. In some embodiments the IV infusion is administered centrally, e.g., using a peripherally inserted central catheter (PICC), a central venous catheter (CVC), a Hickman line, or a portacath device. In some embodiments the endothelial cells (or compositions comprising endothelial cells) are administered to the subjects intraperitoneally, subcutaneously, intra-arterially, or by local injection (for example local injection into the bone marrow).

In some embodiments the effective amount of the endothelial cells is about 2x10⁶ cells per kg bodyweight. In some embodiments the effective amount of the endothelial cells is about 5x10⁶ cells per kg bodyweight. In some embodiments the effective amount of the endothelial cells is about 10x10⁶ cells per kg bodyweight. In some embodiments the effective amount of the endothelial cells is about 15x10⁶ cells per kg bodyweight. In some embodiments the effective amount of the endothelial cells is about 20x10⁶ cells per kg bodyweight. In some embodiments the effective amount of the endothelial cells is about 25x10⁶ cells per kg bodyweight. In some embodiments the effective amount of the endothelial cells is about 30x10⁶ cells per kg bodyweight. In some embodiments the effective amount of the endothelial cells is about 35x10⁶ cells per kg bodyweight. In some embodiments the effective amount of the endothelial cells is about 40x10⁶ cells per kg bodyweight.

In some embodiments the effective amount of the endothelial cells is from about 2x10⁶ cells per kg bodyweight to about 40x10⁶ cells per kg bodyweight. In some embodiments the effective amount of the endothelial cells is from about 2x10⁶ cells per kg bodyweight to about 30x10⁶ cells per kg bodyweight. In some embodiments the effective amount of the endothelial cells is from about 5x10⁶ cells per kg bodyweight to about 30x10⁶ cells per kg bodyweight. In some embodiments the effective amount of the endothelial cells is from about 5x10⁶ cells per kg bodyweight to about 25x10⁶ cells per kg bodyweight. In some embodiments the effective amount of the endothelial cells is from about 5x10⁶ cells per kg bodyweight to about 20x10⁶ cells per kg bodyweight. In some embodiments the effective amount of the endothelial cells is from about 10x10⁶ cells per kg bodyweight to about 20x10⁶ cells per kg bodyweight.

In some embodiments the side effects that are mitigated are of any Grade on the National Cancer Institute's "Common Terminology Criteria for Adverse Events" (NCI-CTCAE) system for grading side effects / adverse events. Such side effects are also referred to as regimen-related toxicities or RRTs.

In some embodiments the side effects that are mitigated are severe side effects (also referred to as severe regimen-related toxicities or SRRTs) - i.e., side effects classified as Grade 3 or greater (i.e. ≥ Grade 3) using the National Cancer Institute's "Common Terminology Criteria for Adverse Events" (NCI-CTCAE) system for grading side effects / adverse events.

In some embodiments the side effects that are mitigated using the methods of the invention are non-hematologic side effects (e.g., non-hematologic RRTs or SRRTs)

In some embodiments the side effects that are mitigated are hematologic side effects (e.g., hematologic RRTs or SRRTs).

As described in the Examples section of this patent disclosure, administration of endothelial cells to human subjects was found to mitigate various non-hematologic side effects of chemotherapy. Accordingly, in some embodiments the non-hematologic side effects that are mitigated using the methods of the invention are oral / gastro-intestinal (GI) side effects. In some embodiments the oral / gastro-intestinal side effects that are mitigated include one or more of mucositis (oral, esophageal, gastric, small intestinal, large intestinal, colonic, rectal or anal), stomatitis, typhlitis, nausea, vomiting, and diarrhea. In some embodiments the non-hematologic side effect that is mitigated is febrile neutropenia. In some embodiments the non-hematologic side effect that is mitigated is infection (e.g., severe infection). In some embodiments the non-hematologic side effects that are mitigated include one or more of pneumonitis, nephritis, neuropathy, chemobrain, cardiotoxicity, engraftment syndrome and/or endothelial dysfunction.

As also described in the Examples section of this patent disclosure, in addition to mitigating various non-hematologic side effects of chemotherapy, administration of endothelial cells to human subjects was also found to improve hematologic / hematopoietic recovery after chemotherapy - for example decreasing the time to hematopoietic recovery (including neutrophil and platelet recovery/engraftment). Accordingly, in some embodiments the present invention provides methods of mitigating hematologic side effects. The hematologic side effects that are mitigated using the methods of the invention can include one or more of thrombocytopenia (low platelet count), neutropenia (low neutrophil count), leukopenia (low white blood cell count), and anemia (low red blood cell count).

These and other embodiments of the invention are described further in other sections of this patent disclosure. In addition, as will be apparent to those of skill in the art, certain modifications and combinations of the various embodiments described herein fall within the scope of the present invention.

### BRIEF DESCRIPTION OF THE FIGURES

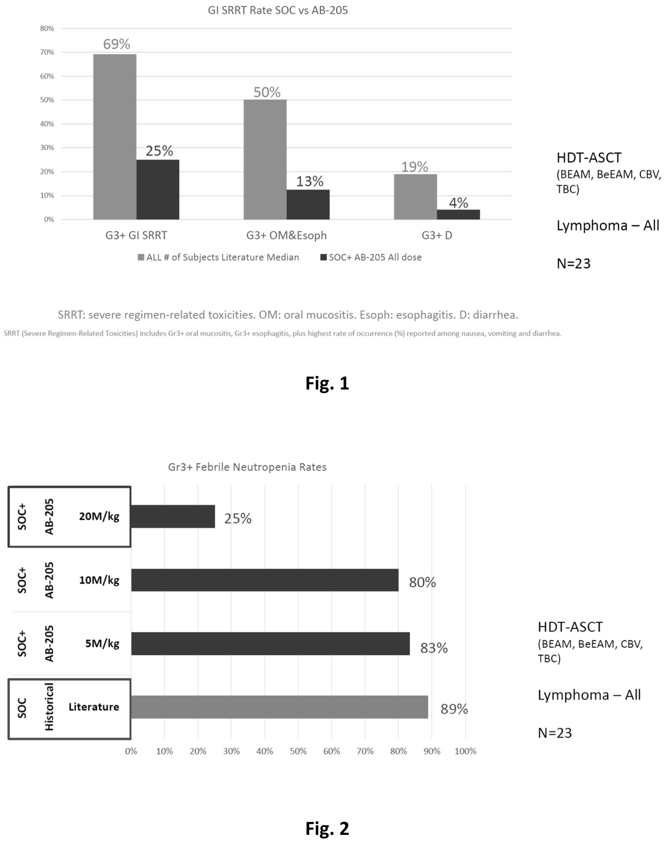

**Fig. 1****.** Data showing that administration of E4ORF1+ HUVECS (AB-205) to human subjects with lymphoma who have undergone high-dose therapy and autologous hematopoietic cell transplant (HDT-AHCT) results in a >50% reduction in oro-gastrointestinal severe regimen-related toxicities (SRRTs) across at all dose levels and administration regimens tested.
**Fig. 2****.** Data showing that administration of E4ORF1+ HUVECS (AB-205) to human subjects with lymphoma who have undergone high-dose therapy and autologous hematopoietic cell transplant (HDT-AHCT) results in a dose-dependent decrease in the incidence of febrile neutropenia across all regimens tested.

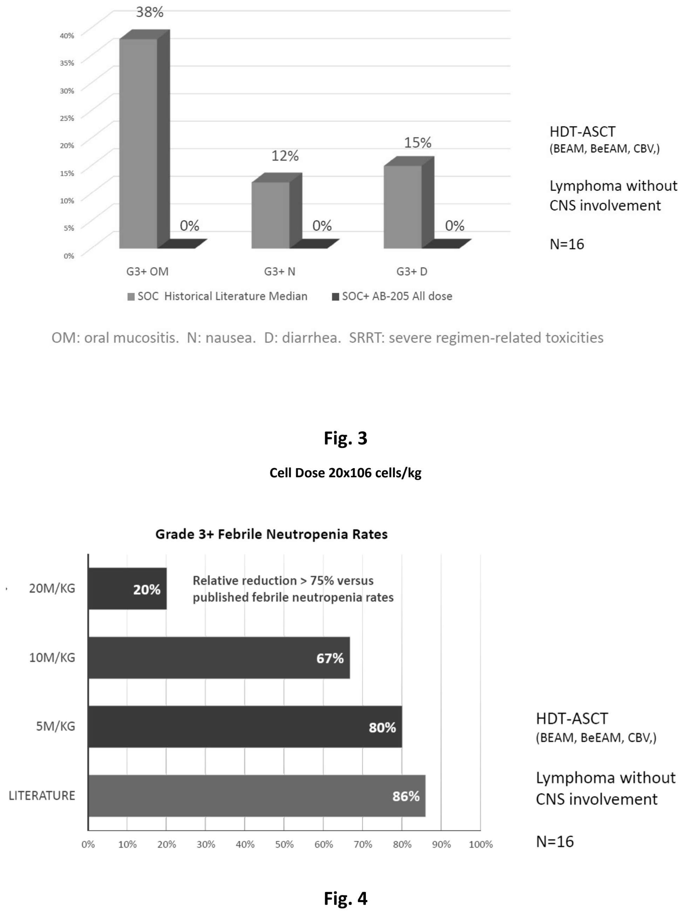

**Fig. 3****.** Data showing that administration of E4ORF1+ HUVECS (AB-205) to human subjects with lymphoma without CNS involvement who have undergone high-dose therapy and autologous hematopoietic cell transplant (HDT-AHCT) results in a 0% rate of oro-gastrointestinal severe regimen-related toxicities (SRRTs) at all dose levels tested.
**Fig. 4****.** Data showing that administration of E4PRF1+ HUVECS (AB-205) to human subjects with lymphoma who have undergone high-dose therapy and autologous hematopoietic cell transplant (HDT-AHCT) results in a greater than 75% reduction of febrile neutropenia (FN).

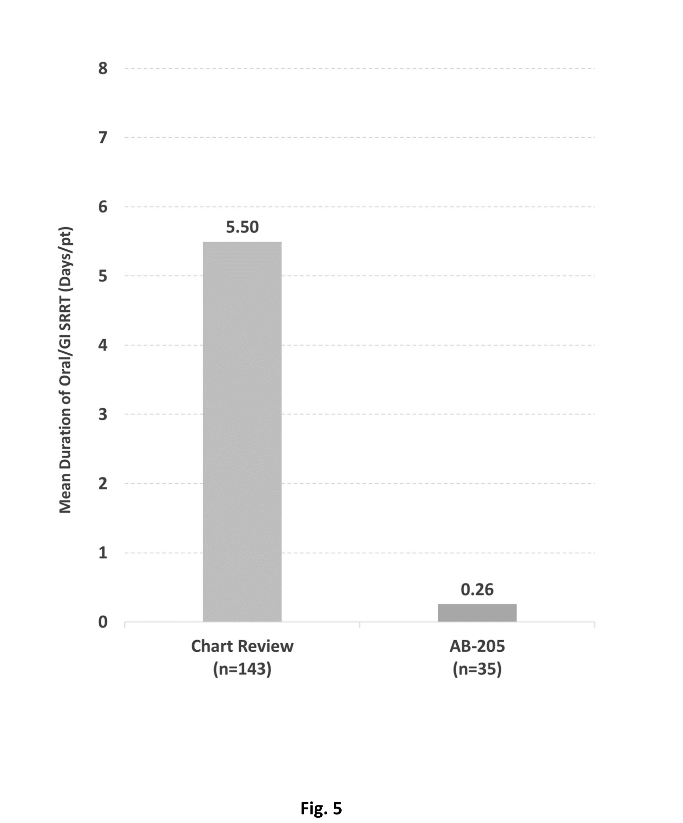

**Fig. 5****.** Data showing that treatment with AB-205 is associated with a substantial reduction in the mean duration of oral/GI severe regimen-related toxicities. Control data from a retrospective chart review is compared to data from patients treated with AB-205. Patients treated with AB-205 (20x10⁶ cells/kg) had a duration of SRRTs that was reduced by 95%. (i.e. from 5.5 days to 0.26 days).

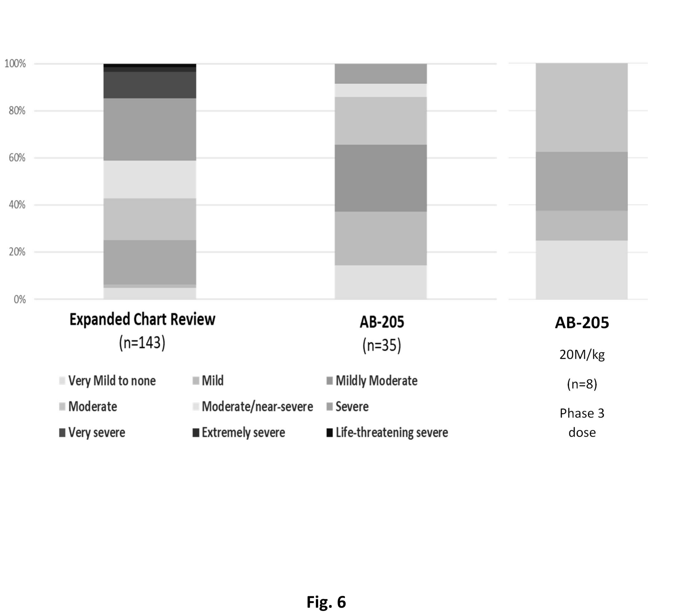

**Fig. 6****.** Data showing that treatment with AB-205 at all doses tested is associated with a decrease in the overall severity of mucositis. Data is shown for all doses (combined) and for the 20x10⁶ cells dose of AB-205. Control data is from a retrospective chart review.

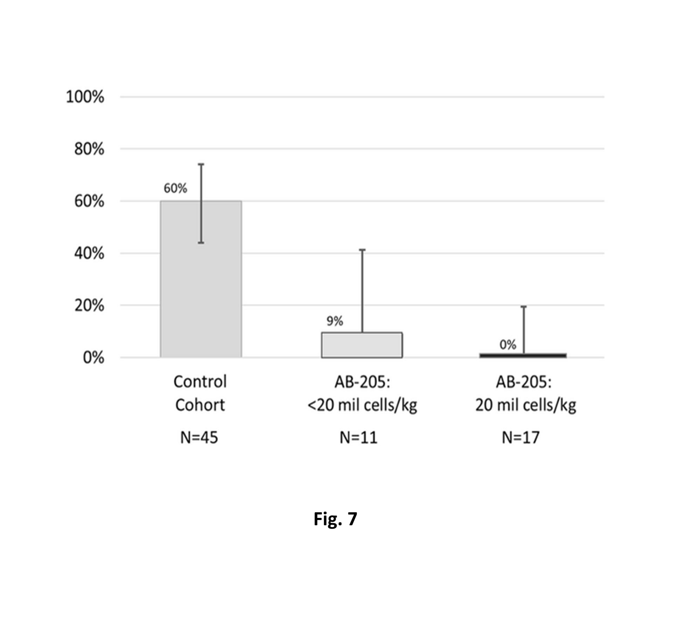

**Fig. 7****.** Data showing effect of AB-205 treatment at doses of less than 20 million cells/kg and at 20 million cells/kg as compared to a cohort control on the rate of severe (CTCAE Grade ≥ 3) regimen-related Oral/GI toxicities (i.e., oral/GI SSRTs). The oral/GI toxicities assessed were: oral mucositis, nausea, vomiting, diarrhea, esophagitis, typhlitis, colitis, and anal mucositis.

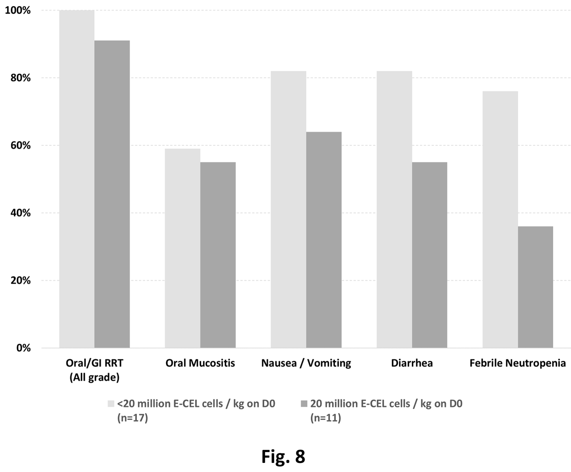

**Fig. 8****.** Data showing that the effect of AB205 treatment on the indicated sub-types of oral / GI SRRTs is dose-dependent. Subjects in this analysis were aged 40 or over.

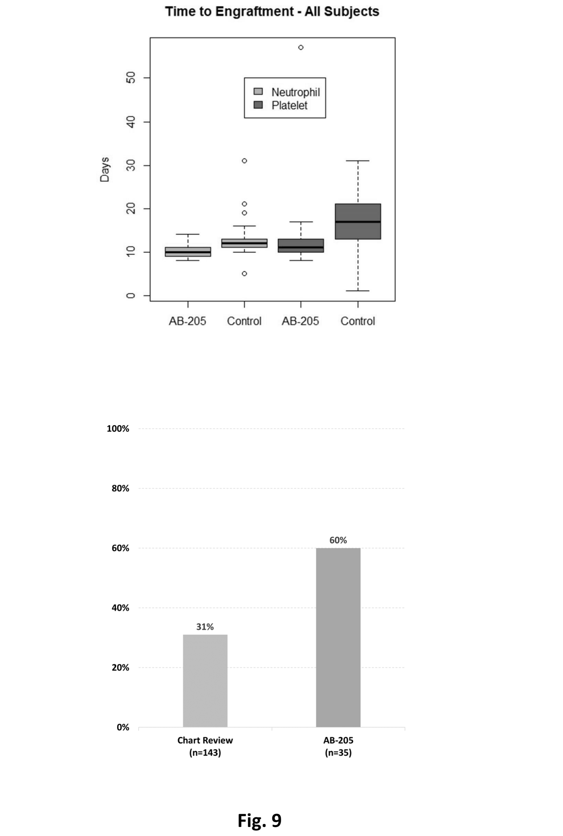

**Fig. 9** Data showing the effects of AB205 treatment hematopoietic recovery (platelet engraftment and neutrophil engraftment). Top panel/graph is data showing time to engraftment for al subjects. Lower panel / graph is showing platelet engraftment within 1 Day of Neutrophil Engraftment.

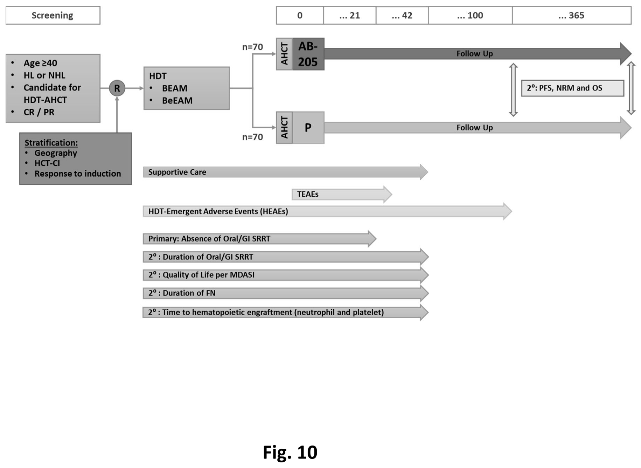

**Fig. 10****.** Schematic representation of a clinical protocol of administration of E4ORF1+ HUVECS (AB-205) to human subjects with lymphoma who have undergone high-dose therapy and autologous hematopoietic cell transplant (HDT-AHCT).

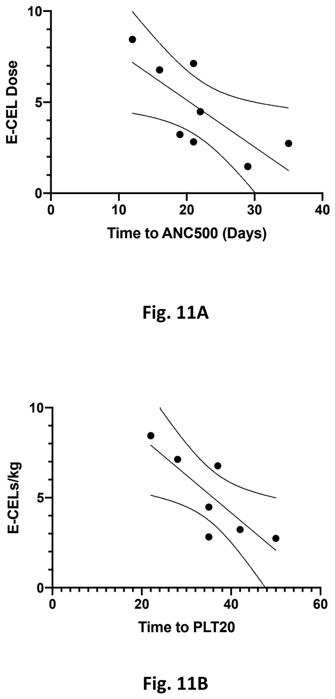

**Fig. 11 A-B**. Data showing that administration of E4ORF1+ HUVECs reduces the time to neutrophil recovery/engraftment **(****Fig. 11A****)** and time to platelet recovery/engraftment **(****Fig. 11B****)** in human subjects with leukemia or myelodysplastic syndrome who have undergone high-dose therapy and allogeneic cord blood stem cell transplant (HDT-AlloSCT). Curves show correlation (solid line) with 95% confidence intervals (dotted lines). For both **Fig. 11A** and **Fig. 11B** the vertical axis is cell dose (E4ORF1+ HUVECs - also referred to as E-Cells) per kg bodyweight. In **Fig. 11A** the horizontal access shows the time to reach an absolute neutrophil count of 500 cells/µL (i.e., ANC500).

### DETAILED DESCRIPTION

The "Summary of the Invention," "Examples," and "Claims" sections of this patent disclosure describe many of the main embodiments of the present invention. This "Detailed Description" section provides certain additional description relating to the compositions and methods of the present invention, and is intended to be read in conjunction with all other sections of this patent disclosure. Furthermore, and as will be apparent to those in the art, the different embodiments described throughout this patent disclosure can be combined in various different way, regardless of any subheadings. Such combinations of the specific embodiments described herein fall within the scope of the present invention

### Definitions

Certain definitions and abbreviations are provided below. Other terms or phrases may be defined elsewhere in this patent disclosure or may have meanings that are clear from the context in which they are used. Unless defined otherwise herein, or unless some other meaning is clear from their use in context herein, all technical and scientific terms and abbreviations used herein have the same meaning as commonly understood by one of ordinary skill in the art to which this invention is related. For example, The Dictionary of Cell and Molecular Biology (5th ed. J.M. Lackie ed., 2013), the Oxford Dictionary of Biochemistry and Molecular Biology (2d ed. R. Cammack et al. eds., 2008), and The Concise Dictionary of Biomedicine and Molecular Biology (2d ed. P-S. Juo, 2002) can provide one of skill with general definitions of some terms used herein.

As used in this specification and the appended claims, the singular forms "a," "an," and "the" include plural referents, unless the context clearly dictates otherwise. The terms "a" (or "an") as well as the terms "one or more" and "at least one" can be used interchangeably.

Furthermore, "and/or" is to be taken as specific disclosure of each of the two specified features or components with or without the other. Thus, the term "and/or" as used in a phrase such as "A and/or B" is intended to include A and B, A or B, A (alone), and B (alone). Likewise, the term "and/or" as used in a phrase such as "A, B, and/or C" is intended to include A, B, and C; A, B, or C; A or B; A or C; B or C; A and B; A and C; B and C; A (alone); B (alone); and C (alone).

Units, prefixes, and symbols are denoted in their Système International de Unites (SI) accepted form. Numeric ranges are inclusive of the numbers defining the range, and any individual value provided herein can serve as an endpoint for a range that includes other individual values provided herein. For example, a set of values such as 1, 2, 3, 8, 9, and 10 is also a disclosure of a range of numbers from 1-10.

Wherever embodiments are described with the language "comprising," otherwise analogous embodiments described in terms of "consisting of" and/or "consisting essentially of" are included.

As used herein, the terms "about" and "approximately," when used in relation to numerical values, mean within + or - 10% of the stated value. In all embodiments where a numerical value is described using the terms "about" or "approximately" the analogous embodiment where the precise numerical value is used is also contemplated. For example, for an embodiment that involves a time of about 24 hours, the analogous embodiment involving a time of 24 hours (i.e., without the "about" qualification) is also contemplated.

As used herein, the term "AB-205" is used to refer to HUVECs engineered to express E4ORF1 (i.e., E4PRF1+ HUVECs) or a composition comprising such cells. Such cells may also be referred to herein as "E-CELs" or "E-CEL UVEC" cells.

As used herein, the abbreviation "AE" refers to adverse event.

As used herein, the abbreviations "AHCT" and "ASCT" refer to autologous hematopoietic stem cell transplantation. The hematopoietic cells used for autologous transplantation are typically obtained from either bone marrow or peripheral blood.

As used herein, the abbreviations "AlloHCT" and "AlloSCT" refer to allogeneic hematopoietic stem cell transplantation. The hematopoietic cells used for allogeneic transplantation are typically obtained from donor bone marrow, peripheral blood, or umbilical cord blood (sometimes referred to as cord blood). For example an AlloSCT procedure can be performed by administering a single umbilical cord blood unit or a double umbilical cord blood unit to a subject.

As used herein, the abbreviation "ANC" refers to absolute neutrophil count.

As used herein, the term "allogeneic" means deriving from, originating in, or being members of the same species, where the members are genetically related or genetically unrelated but genetically similar. For example, in embodiments involving administration of allogeneic hematopoietic cells to a subject, the allogeneic cells are obtained from a donor of the same species as the subject to whom the cells will be administered (i.e. the recipient). In some embodiments the allogeneic cells are obtained from a donor having the same MHC/HLA type as the subject to whom the cells will be administered (i.e. the recipient) - i.e. the donor of the cells and the recipient of the cells are MHC-matched or HLA-matched. In some embodiments, the hematopoietic cells are: (a) obtained from a donor, (b) maintained and/or cultured and/or expanded *ex vivo,* and (c) subsequently administered into a subject of the same species as the donor.

As used herein, the term "autologous" means deriving from or originating in the same subject. For example, in embodiments involving administration of autologous hematopoietic cells to a subject, the autologous cells are obtained from the subject to whom they will be administered (i.e., the donor and recipient of the hematopoietic cells are the same individual). In some embodiments, hematopoietic cells are: (a) obtained from a subject, (b) maintained and/or cultured and/or expanded *ex vivo,* and (c) subsequently administered to the same subject.

As used herein, the abbreviation "BEAM" refers to carmustine ((BCNU)), etoposide, cytarabine (Ara-C, cytosine arabinoside), and melphalan - a combination of chemotherapy. BEAM chemotherapy is a form of high-dose therapy (HDT) and is typically administered prior to hematopoietic stem cell transplantation.

As used herein, the abbreviation "BeEAM" refers to bendamustine, etoposide, cytarabine (Ara-C, cytosine arabinoside), and melphalan - a combination of chemotherapy drugs. BeEAM chemotherapy is a form of high-dose therapy (HDT) and is typically administered prior to hematopoietic stem cell transplantation.

As used herein, the abbreviation "CBV" refers to cyclophosphamide, carmustine (BCNU), and etoposide (VP-16) - a combination of chemotherapy drugs. CBV chemotherapy is a form of high-dose therapy (HDT) and is typically administered prior to hematopoietic stem cell transplantation.

As used herein, the abbreviation "CINV" refers to chemotherapy induced nausea and vomiting.

As used herein, the abbreviation "CR" refers to complete remission.

As used herein, the term "chemotherapy" refers to a cytotoxic pharmaceutical agent, or a therapy involving the administration of a cytotoxic pharmaceutical agent to a subject. Chemotherapy / chemotherapeutic agents are typically used in the treatment of cancer and some other diseases and disorders, such as autoimmune diseases and disorders. In some embodiments the chemotherapy contemplated is high dose chemotherapy (also referred to as high dose therapy or HDT). However, in some embodiments the chemotherapy contemplated is high dose chemotherapy. Similarly, in some embodiments the chemotherapy is myeloblative, while in other embodiments it is not myeloblative. Treatment with chemotherapy typical causes one or more side effects. Side effects of chemotherapy may also be referred to herein as regimen-related toxicities or RRTs. Chemotherapy side effects of NCI-CTCAE Grade 3 or above are considered severe side effects and may be referred to herein as sever regimen-related toxicities or SRRTs.

As used herein, the abbreviation "DLBCL" refers to diffuse large B cell lymphoma.

As used herein, the abbreviation "DMSO" refers to dimethyl sulfoxide.

As used herein, the abbreviation "EC(s)" refers to an endothelial cell(s).

As used herein, the terms "E-CELs" and "E-CEL UVECs" are used to refer to HUVECs engineered to express E4ORF1 (i.e., E4ORF1+ HUVECs) or a composition comprising such cells. Such cells/compositions may also be referred to herein as "AB-205" cells/compositions.

As used herein, the abbreviation "E4ORF" refers to an open reading frame (ORF) of the early 4 (E4) region of an adenovirus genome, or a polypeptide/protein encoded by that ORF (whether the gene or the protein is referred to will be clear from the context of use).

As used herein, the abbreviation "E4ORF1" refers to open reading frame (ORF) 1 of the early 4 (E4) region of an adenovirus genome, or a polypeptide/protein encoded by that ORF (whether the gene or the protein is referred to will be clear from the context of use).

As used herein, the abbreviation "E4ORF1+ HUVECs" is used to refer to HUVECs engineered to express E4ORF1. Such cells contain a recombinant E4ORF1 nucleic acid molecule and express the E4ORF1 protein.

As used herein the term "effective amount" refers to an amount of ECs, or a therapeutic composition comprising ECs, that is sufficient to achieve the stated outcome (e.g. mitigation of a side effect) to a detectable degree or specified degree. In some embodiments the effective amount is specified (e.g., in terms of a number of ECs per kg bodyweight). In other embodiments an appropriate "effective amount" may be determined empirically, for example using standard techniques known in the art, such as dose escalation studies, and may be determined taking into account such factors as the planned route of administration, desired frequency of administration, etc. Furthermore, an "effective amount" may be determined using studies such as those described in the Examples section of this patent disclosure.

The term "engineered" when used in relation to endothelial cells (ECs) refers to ECs cells that have been engineered by man to result in the recited phenotype (e.g., E4ORF1 expression), or to express a recited nucleic acid molecule or polypeptide. The term "engineered cells" is not intended to encompass naturally occurring cells, but is, instead, intended to encompass, for example, cells that comprise a recombinant nucleic acid molecule, or cells that have otherwise been altered artificially, for example so that they express a polypeptide that they would not otherwise express, or so that they express a polypeptide at substantially higher levels than that observed in non-engineered endothelial cells.

As used herein, the term "engraftment syndrome" refers to a clinical condition typically comprising one or more of the following: fever, skin rash, pulmonary edema, weight gain, organ dysfunction, following hematopoietic stem cell transplantation wherein said syndrome occurs at, the time of neutrophil recovery, within about a week prior to neutrophil recovery, or within about two weeks after neutrophil recovery.

The terms "genetic modification" and/or "genetically modified" and/or "gene-modified" refer to any addition, deletion, alteration or disruption of or to a nucleotide sequence or to a cell's genome or to a cell's content of genetic material. In some embodiments, the endothelial cells described herein may, in addition to being genetically modified to provide a nucleic acid molecule that encodes E4ORF1, may also comprise one or more other genetic modifications - as desired. The term "genetic modification" and the above related terms encompass both transient and stable genetic modification and encompass the use of various different gene delivery vehicles and methods including, but not limited to, transduction (viral mediated transfer of nucleic acid to a recipient, either *in vivo* or *in vitro*), transfection (uptake by cells of isolated nucleic acid), liposome mediated transfer and others means of gene delivery that are well known in the art.

As used herein the abbreviation "FN" refers to febrile neutropenia.

As used herein the abbreviation "G-CSF" refers to granulocyte-colony stimulating factor.

As used herein the abbreviation "GI" refers to gastrointestinal.

As used herein the abbreviations "HCT" and "SCT" refer to hematopoietic stem cell transplants or transplantation.

As used herein the abbreviation "HDT" refers to high-dose therapy (i.e., high-dose chemotherapy). The term high dose therapy is widely used in the art and is understood to medical practitioners working in the field of the invention. The term HDT refers to therapy with a dose of a chemotherapeutic agent (or combination of chemotherapeutic agents) that is myeloablative (HDT decreases the number of hematopoietic cells in the bone marrow). HDT also causes numerous other severe regimen related toxicities. HDT is often followed by a hematopoietic stem cell transplant.

As used herein the abbreviation "HL" refers to Hodgkin lymphoma - which is also referred to as Hodgkin's lymphoma.

As used herein the abbreviation "HLA" refers to human leukocyte antigen.

As used herein the abbreviation "HSA" refers to human serum albumin.

As used herein the abbreviation "HUVEC(s)" refers to human umbilical vein endothelial cells.

As used herein the abbreviation "IV" refers to intravenous.

As used herein the abbreviation "LN2" refers to liquid nitrogen.

The term "mitigating" (and grammatical variations thereof) and the phrase "method of mitigating" are used herein in relation to side effects of chemotherapy (e.g. HDT side effects and SRRTs) and refer to producing a permanent or transient reduction in the occurrence, severity, or duration of the side effect. The reduction can range from any detectable reduction in the occurrence, severity or duration of the side effect to total elimination of the side effect. In some embodiments an assessment of the degree of the mitigation (i.e. the degree of the reduction in the occurrence, severity or duration of the side effect) can be made in comparison to a suitable baseline or a suitable control - such as in comparison to the occurrence, severity or duration of the side effect in the same subject after treatment with chemotherapy but before administration of endothelial cells, or in comparison to the occurrence, severity, or duration of the side effect in other individuals whom have been treated similarly to the subject but to whom the endothelial cells have not been administered (e.g. individuals whom have been treated with the same type of chemotherapy as the subject), or in comparison to known data regarding the occurrence, severity, or duration of the side effect in other subjects (e.g. based on published data). In some embodiments, mitigation comprises a reduction in the occurrence, severity, or duration of a side effect of about 2%, 5%, 10%, 20%, 30%, 40%, 50%, 60%, 70%, 80%, 90%, or 100% as compared to one of the suitable baselines or suitable controls mentioned above. In some embodiments the mitigation comprises a reduction in the Grade of the side effects. For example, in some embodiments the mitigation comprises a reduction in the Grade of the side effects as determined using the National Cancer Institute's "Common Terminology Criteria for Adverse Events" (NCI-CTCAE) system for grading side effects / adverse events. In some such embodiments the mitigation comprises a reduction in the Grade of the side effects by 1 point, by 2 points, by 3 points, by 4 points, or by 5 points.

The term "myeloablative" or "myeloablation" refers damage the hematopoietic system and suppression of the ability of the bone marrow to produce blood cells, which typically occurs when hematopoietic cells in the bone marrow are damaged or killed. The term myeloablation encompasses complete myeloablation that destroys the hematopoietic system. It also includes a less than complete myeloablation. Myeloablation may be caused by treatment of a subject with chemotherapy (e.g., HDT) and/or radiation. For example, myeloablation may be caused by a conditioning regimen used to prepare a subject for a subsequent hematopoietic stem cell transplant.

As used herein the abbreviations "NCI-CTCAE" and "CTCAE" refer to the National Cancer Institute's "Common Terminology Criteria for Adverse Events" which is set of criteria for the standardized classification of adverse effects / side effects of drugs used in cancer therapy (*See,* Common Terminology Criteria for Adverse Events (CTCAE) Version 5.0, Published: November 27, 2017, U.S. Department of Health and Human Services, National Institutes of Health, National Cancer Institute; Freites-Martinez et al., Using the Common Terminology Criteria for Adverse Events (CTCAE - Version 5.0) to Evaluate the Severity of Adverse Events of Anticancer Therapies. Actas Dermosifiliogr (Engl Ed). 2021 Jan;112(1):90-92. Grade 1 indicates that the adverse event (AE) is mild, Grade 2 is moderate, Grade 3 is severe (i.e., an severe AE or SAE), Grade 4 is life-threatening, and Grade 5 is fatal (death related to the AE). Table 1, below, provides NCI-CTCAE criteria for Grades 1 through 5 of the side effects/AEs oral mucositis and febrile neutropenia.

**Table 1 - NCI-CTCAE criteria for oral mucositis and febrile neutropenia**

| **AE** | **Definition** | **Grade 1** | **Grade 2** | **Grade 3** | **Grade 4** | **Grade 5** |
|---|---|---|---|---|---|---|
| Mucositis oral | A disorder characterize d by ulceration or inflammatio n of the oral mucosal. | Asymptomat ic or mild symptoms; intervention not indicated | Moderate pain or ulcer that does not interfere with oral intake; modified diet indicated | Severe pain; interfering with oral intake | Life-threatening consequence s; urgent intervention indicated | Death |
| Febrile neutropenia | A disorder characterize d by an ANC <1000/mm3 and a single temperature of >38.3 degrees C (101 degrees F) or a sustained temperature of >=38 degrees C (100.4 degrees F) for more than one hour | - | - | ANC <1000/mm3 with a single temperature of >38.3 degrees C (101 degrees F) or a sustained temperature of >=38 degrees C (100.4 degrees F) for more than one hour | Life-threatening consequence s; urgent intervention indicated | Death |

As used herein the abbreviation "NHL" refers to non-Hodgkin lymphoma (which is also referred to as non-Hodgkin's lymphoma). Non-Hodgkin lymphomas include, but are not limited to Diffuse large B-cell lymphoma (DLBCL), Mantle cell lymphoma (MCL), Lymphoblastic lymphoma, Burkitt lymphoma (BL), Primary mediastinal (thymic) large B-cell lymphoma (PMBCL), Transformed follicular and transformed mucosa-associated lymphoid tissue (MALT) lymphomas, Primary central nervous system (CNS) lymphoma, Acquired immunodeficiency syndrome (AIDS)-associated lymphoma, and Follicular lymphoma (FL).

As used herein the abbreviation "OM" refers to oral mucositis.

As used herein, the term "recombinant" refers to nucleic acid molecules that are isolated, generated and/or designed by man (including by a machine) using methods of molecular biology and genetic engineering (such as molecular cloning), and that either comprise nucleotide sequences that do not exist in nature, or are comprised within nucleotide sequences that do not exist in nature, or are provided in association with nucleotide sequences that they would not be associated with in nature, or that are provided in the absence of nucleotide sequences with which they would ordinarily be associated in nature. Thus, recombinant nucleic acid molecules are to be distinguished from nucleic acid molecules that exist in nature - for example in the genome of an organism. For example, a nucleic acid molecule that comprises a complementary DNA or "cDNA" copy of an mRNA sequence, without any intervening intronic sequences such as would be found in the corresponding genomic DNA sequence, would thus be considered a recombinant nucleic acid molecule. By way of a further example, a recombinant E4ORF1 nucleic acid molecule might comprise an E4ORF1 coding sequence operatively linked to a promoter and/or other genetic elements with which that coding sequence is not ordinarily associated in a naturally-occurring adenovirus genome.

As used herein the abbreviation "RRT" refers to regimen-related toxicity. The terms "RRT" and "chemotherapy side effect" are used interchangeably herein and encompass chemotherapy side effects of any Grade on the NCI-CTCAE grading system. RRTs can include hematologic RRTs and non-hematologic RRTs. Hematologic RRTs can include thrombocytopenia (low platelet count), neutropenia (low neutrophil count), leukopenia (low white blood cell count), and anemia (low red blood cell count). Non-hematologic RRTs can include oral/GI toxicities (including mucositis, oral mucositis, nausea, vomiting and diarrhea), febrile neutropenia (a fever, often with other signs of infection, in a patient with neutropenia), solid organ toxicities (including cardiac, renal, pulmonary and CNS toxicities), veno-occlusive disease, and infections.

As used herein the abbreviation "SRRT" refers to severe regimen-related toxicity. An SRRT is an RRT (i.e., chemotherapy side effect) of Grade 3 or higher on the on the NCI-CTCAE grading system. SRRTs include hematologic SRRTs and non-hematologic SRRTs. Hematologic SRRTs can include thrombocytopenia (low platelet count), neutropenia (low neutrophil count), leukopenia (low white blood cell count), and anemia (low red blood cell count). Non-hematologic SRRTs can include oral/GI toxicities (including mucositis, oral mucositis, nausea, vomiting and diarrhea), febrile neutropenia (a fever, often with other signs of infection, in a patient with neutropenia), solid organ toxicities (including cardiac, renal, pulmonary and CNS toxicities), veno-occlusive disease and infections.

The term "subject" includes mammals - such as humans and non-human primates, as well as other mammalian species including rabbits, rats, mice, cats, dogs, horses, cows, sheep, goats, pigs and the like. In some embodiments the subjects are mammalian subjects. In some embodiments the subjects are non-human primates. In some embodiments the subjects are humans. When the subjects are human the terms "subject" and "patient" may be used interchangeably.

As used herein the abbreviation "TBC" refers to thiotepa, busulfan, and cyclophosphamide - a combination of chemotherapy drugs. TBC chemotherapy is a form of high-dose therapy (HDT) that is CNS penetrant and is typically administered prior to hematopoietic stem cell transplantation.

As used herein the abbreviation "TEAE" refers to treatment-emergent adverse event.

As used herein the abbreviation "UVEC(s)" refers to umbilical vein endothelial cells.

As used herein the abbreviation "WBC" refers to white blood cell.

### Endothelial Cells

In some embodiments the endothelial cells (ECs) described herein are any suitable type of vascular endothelial cells known in the art. In some embodiments the endothelial cells are primary endothelial cells. In some embodiments the endothelial cells are mammalian cells, such as human or non-human primate cells, or rabbit, rat, mouse, goat, pig, or other mammalian cells. In some embodiments the endothelial cells are primary human endothelial cells. In some embodiments the endothelial cells are umbilical vein endothelial cells (UVECs), such as human umbilical vein endothelial cells (HUVECs). In some embodiments the endothelial cells are adipose ECs. In some embodiments the endothelial cells are skin ECs. In some embodiments the endothelial cells are cardiac ECs. In some embodiments the endothelial cells are kidney ECs. In some embodiments the endothelial cells are lung ECs. In some embodiments the endothelial cells are liver ECs. In some embodiments the endothelial cells are bone marrow ECs. In some embodiments the endothelial cells are autologous ECs. In some embodiments the endothelial cells are allogeneic ECs. Other suitable endothelial cells that can be used include those described in U.S. Patent No. 8,465,732, the contents of which are hereby incorporated by reference.

### E4ORF1

Several of the embodiments of the present invention involve engineered endothelial cells (ECs) that are E4ORF1+ - i.e., ECs cells that express an E4ORF1 polypeptide. The "E4ORF1" polypeptide is encoded by open reading frame (ORF) 1 of the early 4 (E4) region of the adenovirus genome. E4ORF1+ ECs for use in accordance with the present invention typically comprise a recombinant nucleic acid molecule that contains an E4ORF1 coding sequence operatively linked to a promoter suitable for expression of the E4ORF1 coding sequence in endothelial cells.

E4ORF1 amino acid sequences and nucleotide sequences are known in the art. Any such sequences may be used in accordance with the present invention. In some embodiments the E4ORF1 polypeptide may be from any suitable adenovirus type or strain, such as human adenovirus type 2, 3, 5, 7, 9, 11, 12, 14, 34, 35, 46, 50, or 52. In some preferred embodiments the polypeptide sequence used is from human adenovirus type 5. Amino acid sequences of such adenovirus polypeptides, and nucleic acid sequences that encode such polypeptides, are well known in the art and available in well-known publicly available databases, such as the Genbank database. For example, suitable sequences include the following: human adenovirus 9 (Genbank Accession No. CAI05991), human adenovirus 7 (Genbank Accession No. AAR89977), human adenovirus 46 (Genbank Accession No. AAX70946), human adenovirus 52 (Genbank Accession No. ABK35065), human adenovirus 34 (Genbank Accession No. AAW33508), human adenovirus 14 (Genbank Accession No. AAW33146), human adenovirus 50 (Genbank Accession No. AAW33554), human adenovirus 2 (Genbank Accession No. AP.sub.--000196), human adenovirus 12 (Genbank Accession No. AP.sub.--000141), human adenovirus 35 (Genbank Accession No. AP.sub.-000607), human adenovirus 7 (Genbank Accession No. AP.sub.--000570), human adenovirus 1 (Genbank Accession No. AP.sub.--000533), human adenovirus 11 (Genbank Accession No. AP.sub.--000474), human adenovirus 3 (Genbank Accession No. ABB 17792), and human adenovirus type 5 (Genbank accession number D12587). In one embodiment the E4ORF1 sequence used is that having NCBI accession number AZR66741.1. In one embodiment the E4ORF1 sequence used is that having NCBI accession number AP_000232.1. In one embodiment the E4ORF1 sequence used is has the amino acid sequence
MAAAVEALFVVLEREGAILPRQEGFSGVYVFFSPINFVIPPMGAVMLSLRLRVCIPPG YFGRFLALTDVNQPDVFTESYIMTPDMTEELSVVLFNHGDQFFYGHAGMAVVRLML IRVVFPVVRQASNV (SEQ ID NO. 1).

In some embodiments the E4ORF1 polypeptide may have amino acid sequence that is, or be encoded by a nucleic acid sequence that is, a variant, derivative, mutant, or fragment of any of the specific sequences provided herein or known in the art provided that such variants, derivatives, mutants, or fragments are, or encode, a polypeptide that has one or more of the functional properties of adenovirus E4ORF1 described herein. In some embodiments, the variants, derivatives, mutants, or fragments have about an 85% identity to the known sequence, or about an 88%, 89%, 90%, 91%, 92%, 93%, 94%, 95%, 96%, 97%, 98%, or 99% sequence identity to the known sequence. In some embodiments, a variant, derivative, mutant, or fragment of a known nucleotide sequence is used that varies in length by about 50 nucleotides, or about 45 nucleotides, or about 40 nucleotides, or about 35 nucleotides, or about 30 nucleotides, or about 28 nucleotides, 26 nucleotides, 24 nucleotides, 22 nucleotides, 20 nucleotides, 18 nucleotides, 16 nucleotides, 14 nucleotides, 12 nucleotides, 10 nucleotides, 9 nucleotides, 8 nucleotides, 7 nucleotides, 6 nucleotides, 5 nucleotides, 4 nucleotides, 3 nucleotides, 2 nucleotides, or 1 nucleotide relative to the known nucleotide sequence. In some embodiments, a variant, derivative, mutant, or fragment of a known amino sequence is used that varies in length about 50 amino acids, or about 45 amino acids, or about 40 amino acids, or about 35 amino acids, or about 30 amino acids, or about 28 amino acids, 26 amino acids, 24 amino acids, 22 amino acids, 20 amino acids, 18 amino acids, 16 amino acids, 14 amino acids, 12 amino acids, 10 amino acids, 9 amino acids, 8 amino acids, 7 amino acids, 6 amino acids, 5 amino acids, 4 amino acids, 3 amino acids, 2 amino acids, or 1 amino acid relative to the known amino acid sequence.

In some embodiments E4ORF1 sequences are used without other sequences from the adenovirus E4 region - for example not in the context of the entire E4 region or not together with other ORFs in the E4 region. However, in other embodiments E4ORF1 may be used in conjunction with one or more other ORFs from the E4 region, such as E4ORF2, E4ORF3, E4ORF4, E4ORF5 or E4ORF6/7 sequences. For example, although E4ORF1 sequences can be used in constructs (such as a viral vectors) that contain other sequences, genes, or coding regions (such as promoters, marker genes, antibiotic resistance genes, and the like), in certain embodiments, the E4ORF1 sequences are used in constructs that do not contain the entire E4 region, or that do not contain other ORFs from the entire E4 region, such as E4ORF2, E4ORF3, E4ORF4, and/or E4ORF5.

E4ORF1 encoding sequences can be present in constructs or vectors that contain various other sequences, genes, or coding regions, for example, promoters, enhancers, antibiotic resistance genes, reporter genes or expression tags (such as, for example nucleotides sequences encoding GFP), or any other nucleotide sequences or genes that might be desirable.

E4ORF1-encoding nucleic acid molecules can be under the control of one or more promoters to allow for expression. Any promoter able to drive expression of the E4ORF1 nucleic acid sequences in endothelial cells can be used. Examples of suitable promoters include, but are not limited to, the CMV, SV40, RSV, HIV-Ltr, and MML promoters. The promoter can also be a promoter from the adenovirus genome, or a variant thereof. For example, in some embodiments the promoter may be a promoter that drives expression of E4ORF1 in nature in an adenovirus genome. However, in other embodiments the promoter is not one that drives expression of E4ORF1 in nature in an adenovirus genome.

The E4ORF1-encoding sequences may comprise naturally occurring nucleotides, synthetic nucleotides, or a combination thereof. For example, in some embodiments the nucleic acid molecules of the invention can comprise RNA, such as synthetic modified RNA that is stable within cells and can be used to direct protein expression/production directly within cells. In other embodiments the E4ORF1-encoding sequences can comprise DNA. In embodiments where DNA is used, the DNA sequences may be operably linked to one or more suitable promoters and/or regulatory elements to allow (and/or facilitate, enhance, or regulate) expression within cells, and may be present in one or more suitable vectors or constructs.

The E4ORF1-encoding sequences can be introduced into endothelial cells using any suitable system known in the art, including, but not limited to, transfection techniques and viral-mediated transduction techniques. Transfection methods that can be used in accordance with the present invention include, but are not limited to, liposome-mediated transfection, polybrene-mediated transfection, DEAE dextran-mediated transfection, electroporation, calcium phosphate precipitation, microinjection, and micro-particle bombardment. Viral-mediated transduction methods that can be used include, but are not limited to, lentivirus-mediated transduction, adenovirus-mediated transduction, retrovirus-mediated transduction, adeno-associated virus-mediated transduction and herpesvirus-mediated transduction.

In some embodiments the E4ORF1-encoding sequences are in a vector. In some embodiments the E4ORF1-encoding sequences are in a viral vector. In some embodiments the E4ORF1-encoding sequences are in a lentiviral vector. In some embodiments the E4ORF1-encoding sequences are in an adenoviral vector. In some embodiments the E4ORF1-encoding sequences are in adeno-associated virus vector. In some embodiments the E4ORF1-encoding sequences are in a retroviral vector. In some embodiments the E4ORF1-encoding sequences are in a Moloney murine leukemia virus (MMLV) vector (a type of retroviral vector).

In some embodiments the compositions of the present invention (i.e., therapeutic compositions) comprise both E4ORF1+ and E4ORF1-negative endothelial cells (as described in the Example, the E4ORF1+ HUVECs used in carrying out the clinical trials contained both E4ORF1+ E4ORF1-negative endothelial cells). In some embodiments at least about 75% of the endothelial cells in the therapeutic composition are E4ORF1+. In some embodiments at least about 80% of the endothelial cells in the therapeutic composition are E4ORF1+. In some embodiments at least about 85% of the endothelial cells in the therapeutic composition are E4ORF 1+. In some embodiments at least about 90% of the endothelial cells in the therapeutic composition are E4ORF1+. In some embodiments at least about 95% of the endothelial cells in the therapeutic composition are E4ORF1+. In some embodiments at least about 98% of the endothelial cells in the therapeutic composition are E4ORF1+. In some embodiments at least about 99% of the endothelial cells in the therapeutic composition are E4ORF1+.

As described in the Examples, the E4ORF1+ HUVECs used in carrying out the clinical trials contained from about 0.88 to about 1.4 copies of an integrated E4ORF1 coding sequence per cell E4ORF1. In some embodiments the compositions of the present invention (i.e., therapeutic compositions) comprise endothelial cells that, on average across all of the endothelial cells in the composition, comprise less than one copy of a genomically integrated E4ORF1 coding sequence per cell. In some embodiments the compositions of the present invention (i.e., therapeutic compositions) comprise endothelial cells that, on average across all of the endothelial cells in the composition, comprise about on copy of a genomically integrated E4ORF1 coding sequence per cell. In some embodiments the compositions of the present invention (i.e., therapeutic compositions) comprise endothelial cells that, on average across all of the endothelial cells in the composition, comprise more than one copy of a genomically integrated E4ORF1 coding sequence per cell. In some embodiments the compositions of the present invention (i.e., therapeutic compositions) comprise endothelial cells that, on average across all of the endothelial cells in the composition, comprise about 0.7 copies of a genomically integrated E4ORF1 coding sequence per cell. In some embodiments the compositions of the present invention (i.e., therapeutic compositions) comprise endothelial cells that, on average across all of the endothelial cells in the composition, comprise about 0.8 copies of a genomically integrated E4ORF1 coding sequence per cell. In some embodiments the compositions of the present invention (i.e., therapeutic compositions) comprise endothelial cells that, on average across all of the endothelial cells in the composition, comprise about 0.9 copies of a genomically integrated E4ORF1 coding sequence per cell. In some embodiments the compositions of the present invention (i.e., therapeutic compositions) comprise endothelial cells that, on average across all of the endothelial cells in the composition, comprise about 1.0 copies of a genomically integrated E4ORF1 coding sequence per cell. In some embodiments the compositions of the present invention (i.e., therapeutic compositions) comprise endothelial cells that, on average across all of the endothelial cells in the composition, comprise about 1.1 copies of a genomically integrated E4ORF1 coding sequence per cell. In some embodiments the compositions of the present invention (i.e., therapeutic compositions) comprise endothelial cells that, on average across all of the endothelial cells in the composition, comprise about 1.2 copies of a genomically integrated E4ORF1 coding sequence per cell. In some embodiments the compositions of the present invention (i.e., therapeutic compositions) comprise endothelial cells that, on average across all of the endothelial cells in the composition, comprise about 1.3 copies of a genomically integrated E4ORF1 coding sequence per cell. In some embodiments the compositions of the present invention (i.e., therapeutic compositions) comprise endothelial cells that, on average across all of the endothelial cells in the composition, comprise about 1.4 copies of a genomically integrated E4ORF1 coding sequence per cell. In some embodiments the compositions of the present invention (i.e., therapeutic compositions) comprise endothelial cells that, on average across all of the endothelial cells in the composition, comprise about 1.5 copies of a genomically integrated E4ORF1 coding sequence per cell.

In some embodiments the presence of E4ORF1 coding sequences can be confirmed and/or quantified using standard nucleic acid detection and/or quantification assays known in the art, such as PCR-based techniques (e.g., quantitative PCR) and sequencing-based techniques (e.g., quantitative next generation sequencing-based techniques). In some embodiments the presence of E4ORF1 polypeptides can be confirmed and/or quantified using standard protein detection and/or quantification assays known in the art, such as antibody-based techniques. In some embodiments the expression of functional E4ORF1 polypeptide (or an appropriate amount of functional E4ORF1 polypeptide can be confirmed and/or quantified using functional assays (e.g., in vitro or in vivo assays) for any of the functional properties of E4ORF1-expressing endothelial cells that are known in the art (such as any of those described in U.S. Patent No. 8,465,732) or described in the Examples section of this patent disclosure. In some of such embodiments the results of any of such assays can be compared between batches of E4ORF1+ endothelial cells (e.g. between a test batch and a control batch having known E4ORF1 properties), for example to assess consistency and/or to make any adjustments based thereon.

The handling, manipulation, and expression of E4ORF1 sequences in endothelial cells may be performed using conventional techniques of molecular biology and cell biology. Such techniques are well known in the art. For example, one may refer to the teachings of Sambrook, Fritsch and Maniatis eds., "Molecular Cloning A Laboratory Manual, 2nd Ed., Cold Springs Harbor Laboratory Press, 1989); the series Methods of Enzymology (Academic Press, Inc.), or any other standard texts for guidance on suitable techniques to use in handling, manipulating, and expressing nucleotide and/or amino acid sequences. Additional aspects relevant to the handling and expression of E4ORF1 sequences in endothelial cells are described in U.S. Patent No. 8,465,732, the contents of which are hereby incorporated by reference.

### Therapeutic Compositions

As used herein the term "therapeutic composition" refers to a composition suitable for administration to a human subject. The terms "therapeutic composition" and "composition" may be used interchangeably herein - as will be clear from the context of use. For example, where a composition is referred to in the context of administration to a human subject, the composition is necessarily a therapeutic composition.

In some embodiments the present invention provides therapeutic compositions comprising an effective amount of the specified endothelial cells (e.g. E4ORF1+ HUVECs) in solution suitable for administration to a human subject. Such compositions typically comprise the specified endothelial cells in physiological saline solution. In some embodiments the compositions may comprise one or more additional excipients suitable for administration to human subjects and compatible with cell therapies, such as buffers, salts, preservatives, polysaccharides (e.g., dextrans), proteins (e.g., albumin) and the like. In some embodiments the compositions may comprise a low concentration of a cryopreservative (importantly, the Phase 1 studies shown in the Examples section of this patent disclosure demonstrated that therapeutic compositions comprising E4ORF1+ HUVECs and low concentrations of the cryopreservative DMSO can be safely administered to human subjects). In some embodiments the therapeutic compositions of the present invention comprise Dextran 40. In some embodiments the therapeutic compositions of the present invention comprise human serum albumin (HSA). In some embodiments the therapeutic compositions of the present invention comprise dimethyl sulfoxide (DMSO). In some embodiments the therapeutic compositions of the present invention comprise E4ORF1+ HUVEC cells, Dextran40, HSA and DMSO in a physiological saline. In some such embodiments such compositions comprise E4ORF1+ HUVEC cells at a concentration of about 5x10⁶ cells per ml. In some such embodiments such compositions comprise Dextran 40 at a concentration of about 8mg/ml. In some such embodiments such compositions comprise HSA at a concentration of about 4.5 mg/ml. In some such embodiments such compositions comprise DMSO at a concentration of about 0.25 mg/ml.

Typically, the endothelial cells (e.g., E4PRF1+ HUVECs) for use in the methods of the present invention are supplied (e.g., to the medical facility where there will be administered to subjects) in frozen form - i.e. in a frozen composition containing the specified endothelial cells (e.g. E4ORF1+ HUVECs) and a cryopreservative. The frozen compositions are thawed prior to use, for example using standard methods known in the art. In some embodiments the frozen composition may be thawed and then administered to a subject without dilution. However, in other embodiments the frozen composition is thawed and then diluted in a suitable diluent to result in the generation of a therapeutic composition containing the desired or specified concentration cells and the desired or specified concentrations of excipients, for example as described above.

In some embodiments the present invention provides a kit comprising a therapeutic composition according to the present invention in sterile packaging and instructions for administering the therapeutic composition to a subject.

In some embodiments the present invention provides a kit comprising a cryopreserved composition comprising endothelial cells according to the present invention (e.g., E4ORF1+ HUVECs) in sterile packaging and instructions for preparing a therapeutic composition from the cryopreserved composition and administering the therapeutic composition to a subject.

### Administration

The present invention provides methods that involve administration of effective amounts of endothelial cells, or therapeutic compositions comprising endothelial cells, to subjects.

Any suitable route of administration may be used. In some embodiments the endothelial cells (or compositions comprising endothelial cells) are administered to the subjects intravenously, for example by (IV) infusion. In some embodiments the IV infusion is administered peripherally, e.g., using a peripheral IV catheter. In some embodiments the IV infusion is administered centrally, e.g., using a peripherally inserted central catheter (PICC), a central venous catheter (CVC), a Hickman line, or a portacath device.

Any suitable timing of administration or rate of administration or administration regimen may be used such that the effective amount of the endothelial cells is delivered to the subject.

In some embodiments the effective amount of the endothelial cells (or composition comprising the endothelial cells) is administered to a subject in a single administration (i.e., in a single administration/dosing regimen). In some embodiments the effective amount of the endothelial cells (or composition comprising the endothelial cells) is divided into two or more doses to be administered to the subject separately in multiple administrations delivered at different times (i.e., in a multiple administration/dosing regimen). The separate doses in such a multiple administration regimen can be separated by any suitable amount of time. In some embodiments the separate doses in such a multiple administration regimen are administered 1 day apart, or 2 days or apart, or 3 days apart.

In some embodiments, the effective amount of endothelial cells is administered to the subject by IV infusion over a desired period of time. In some embodiments the effective amount is administered to the subject by IV infusion over a period of about 1 hour. In some embodiments the effective amount is administered to the subject by IV infusion at a rate of about 6ml/minute. In some embodiments the effective amount of endothelial cells is administered to the subject by IV infusion at a rate of about 30x10⁶ cells/minute.

In some embodiments the endothelial cells (or compositions comprising endothelial cells) are first administered to the subjects from 0 to 28 days after the subject first exhibits side effects from the chemotherapy. In some embodiments the endothelial cells (or compositions comprising endothelial cells) are first administered to the subjects from 0 to 7 days after the subject first exhibits side effects from the chemotherapy. In some embodiments the endothelial cells (or compositions comprising endothelial cells) are first administered to the subjects from 0 to 2 days after the subject first exhibits side effects from the chemotherapy. In some embodiments the endothelial cells (or compositions comprising endothelial cells) are first administered to the subjects from 0 to 1 days after the subject first exhibits side effects from the chemotherapy. In some embodiments the endothelial cells (or compositions comprising endothelial cells) are first administered to the subjects before the subject first exhibits side effects from the chemotherapy.

In some embodiments the endothelial cells (or compositions comprising endothelial cells) are first administered to the subjects from 0 to 28 days after the subject completes treatment with the chemotherapy. In some embodiments the endothelial cells (or compositions comprising endothelial cells) are first administered to the subjects from 0 to 7 days after the subject completes treatment with the chemotherapy. In some embodiments the endothelial cells (or compositions comprising endothelial cells) are first administered to the subjects from 0 to 2 days after the subject completes treatment with the chemotherapy. In some embodiments the endothelial cells (or compositions comprising endothelial cells) are first administered to the subjects from 0 to 1 days after the subject completes treatment with the chemotherapy.

In some such embodiments the subject receives a hematopoietic stem cell transplant at the same time that the endothelial cells are administered to the subject. In some such embodiments the hematopoietic stem cell transplant and the endothelial cells are administered to the subject together - for example in the same intravenous (IV) infusion.

In some embodiments the endothelial cells are administered to the subject after the subject receives a hematopoietic cell transplant. In some such embodiments the endothelial cells are administered to the subject on the same day that subject receives a hematopoietic cell transplant i.e., on Day 0, where Day 0 is the day of the hematopoietic cell transplant). In some such embodiments the endothelial cells are administered to the subject one day after the subject receives a hematopoietic cell transplant (i.e., on Day 1). In some such embodiments the endothelial cells are administered to the subject two days after the subject receives a hematopoietic cell transplant (i.e., on Day 2). In some such embodiments the endothelial cells are administered to the subject three days after the subject receives a hematopoietic cell transplant (i.e., on Day 3). In some such embodiments the endothelial cells are administered to the subject about 2 hours after that subject receives a hematopoietic cell transplant. In some such embodiments the endothelial cells are administered to the subject about 3 hours after the subject receives a hematopoietic cell transplant. In some such embodiments the endothelial cells are administered to the subject about 4 hours after the subject receives a hematopoietic cell transplant. In some such embodiments the endothelial cells are administered to the subject about 6 hours after the subject receives a hematopoietic cell transplant. In some such embodiments the endothelial cells are administered to the subject about 8 hours after the subject receives a hematopoietic cell transplant. In some such embodiments the endothelial cells are administered to the subject about 10 hours after the subject receives a hematopoietic cell transplant. In some such embodiments the endothelial cells are administered to the subject about 12 hours after the subject receives a hematopoietic cell transplant.

In some embodiments the endothelial cells are administered to the subject before the subject receives a hematopoietic cell transplant. In some such embodiments the endothelial cells are administered to the subject one day before the subject receives a hematopoietic cell transplant (i.e., on Day -1). In some such embodiments the endothelial cells are administered to the subject two days before the subject receives a hematopoietic cell transplant (i.e., on Day -2). In some such embodiments the endothelial cells are administered to the subject three days before the subject receives a hematopoietic cell transplant (i.e., on Day -3). In some such embodiments the endothelial cells are administered to the subject about 2 hours before that subject receives a hematopoietic cell transplant. In some such embodiments the endothelial cells are administered to the subject about 3 hours before the subject receives a hematopoietic cell transplant. In some such embodiments the endothelial cells are administered to the subject about 4 hours before the subject receives a hematopoietic cell transplant. In some such embodiments the endothelial cells are administered to the subject about 6 hours before the subject receives a hematopoietic cell transplant. In some such embodiments the endothelial cells are administered to the subject about 8 hours before the subject receives a hematopoietic cell transplant. In some such embodiments the endothelial cells are administered to the subject about 10 hours before the subject receives a hematopoietic cell transplant. In some such embodiments the endothelial cells are administered to the subject about 12 hours before the subject receives a hematopoietic cell transplant.

In some such embodiments the endothelial cells are administered to the subject within (i.e., either before or after) one day of the hematopoietic cell transplant. In some such embodiments the endothelial cells are administered to the subject within two days of the hematopoietic cell transplant. In some such embodiments the endothelial cells are administered to the subject within three days of the hematopoietic cell transplant. In some such embodiments the endothelial cells are administered to the subject within about 2 hours of the hematopoietic cell transplant. In some such embodiments the endothelial cells are administered to the subject within about 3 hours of the hematopoietic cell transplant. In some such embodiments the endothelial cells are administered to the subject within about 4 hours of the hematopoietic cell transplant. In some such embodiments the endothelial cells are administered to the subject within about 6 hours of the hematopoietic cell transplant. In some such embodiments the endothelial cells are administered to the subject within about 8 hours of the hematopoietic cell transplant. In some such embodiments the endothelial cells are administered to the subject within about 10 hours of the hematopoietic cell transplant. In some such embodiments the endothelial cells are administered to the subject within about 12 hours of the hematopoietic cell transplant.

### Hematopoietic Stem Cell Transplants

Hematopoietic stem cell transplants are performed using standard methods that are well known in the art.

For example, methods for performing autologous hematopoietic cell transplantation (AHCT) are well known in the art and can readily be performed by medical professionals using well known methodologies and using autologous hematopoietic cells obtained using well known methodologies. Examples of such methodologies are described in Dahi, P.B., et al., Strategies to improve outcomes of autologous hematopoietic cell transplant in lymphoma. Bone Marrow Transplant, 2019. 54(7): p. 943-960, Gyurkocza & Sandmaier, Conditioning regimens for hematopoietic cell transplantation: one size does not fit all. Blood, 2014. 124(3): p. 344-53, Loke et al., Outcomes of EAM conditioned autologous haematopoietic SCT for lymphoma. A matched pairs retrospective single-centre study analysis, Bone Marrow Transplantation (2013) 48, 1486-1487, the contents of which are hereby incorporated by reference.

Similarly, methods for performing allogeneic hematopoietic stem cell transplantation (AlloSCT) are well known in the art and can readily be performed by medical professionals using well known methodologies and using allogeneic cord blood stem cells obtained using well known methodologies. Examples of such methodologies are described *in* Bacigalupo et al., Defining the intensity of conditioning regimens working definitions, Biol Blood Marrow Transplant. 2009; 15(12): 1628-1633, Gyurkocza & Sandmaier, Conditioning regimens for hematopoietic cell transplantation: one size does not fit all. Blood, 2014. 124(3): p. 344-53, Chaudhry et al., The Incidence and Severity of Oral Mucositis among Allogeneic Hematopoietic Stem Cell Transplantation Patients: A Systematic Review, Biol Blood Marrow Transplant 22 (2016) 605e616, Eapen et al., "Mismatched Related and Unrelated Donors for Allogeneic Hematopoietic Cell Transplantation for Adults with Hematologic Malignancies, Biol Blood Marrow Transplant 20 (2014) 1485e1492.

### High Dose Therapy

High Dose Therapy (HDT) is performed using methods and chemotherapeutic agents that are well known in the art.

For example, numerous methods for performing HDT therapy for the treatment of lymphomas and leukemias (as well as other conditions) are well known in the art and can readily be performed by medical professionals using well known methodologies and using well known chemotherapeutic agents or combinations of chemotherapeutic agents. Some examples of suitable HDT methods and agents are provided in the Examples section of this patent disclosure (i.e., those used in the clinical trials described in the Examples). Additional examples of suitable methodologies and agents and are described in Philip, T., et al., High-dose therapy and autologous bone marrow transplantation after failure of conventional chemotherapy in adults with intermediate-grade or high-grade non-Hodgkin's lymphoma. N Engl J Med, 1987. 316(24): p. 1493-8 and Philip, T., et al., Autologous bone marrow transplantation as compared with salvage chemotherapy in relapses of chemotherapy-sensitive non-Hodgkin's lymphoma. N Engl J Med, 1995. 333(23): p. 1540-5, Gyurkocza & Sandmaier, Conditioning regimens for hematopoietic cell transplantation: one size does not fit all. Blood, 2014. 124(3): p. 344-53, Loke et al., Outcomes of EAM conditioned autologous haematopoietic SCT for lymphoma. A matched pairs retrospective single-centre study analysis, Bone Marrow Transplantation (2013) 48, 1486-1487.

### Adjunctive Therapies

A variety of adjunctive therapeutic methods or adjunctive agents may be used in conjunction with the methods and compositions described herein. For example, such adjunctive treatments may include treatment of subjects with radiation, treatment of subjects with chemotherapeutic agents, treatment of subjects with immunotherapies (such as checkpoint inhibitors - e.g. Keytruda^{®}), treatment of subjects with antibodies (e.g. Heceptin^{®}), treatment of subjects with cell therapies, treatment of subjects with surgery, treatment of subjects with growth factors, cytokines, anti-inflammatory agents, anti-bacterial agents, anti-fungal agents, anti-emetics, anti-diarrheal agents, granulocyte colony-stimulating factor, granulocyte-macrophage colony-stimulating factor, erythropoietic agents, anti-arrhythmic agents, and the like. Any treatment useful for the treatment of the subject's underlying conditions (e.g., cancer), or for the treatment of side effects of chemotherapy in the subject, may be used in conjunction with the methods described herein. Examples of suitable "standard of care" supportive therapies useful for the care of subjects that have undergone HDT are provided in Example 4.

The invention is further described with reference to the following non-limiting Examples.

### EXAMPLES

### Introduction to Examples

High-dose therapy and autologous hematopoietic cell transplantation (HDT-AHCT) is a standard-of-care therapy for patients with aggressive systemic non-Hodgkin (NHL) and Hodgkin lymphomas (HL) and is administered with curative intent (Philip et al., "High-dose therapy and autologous bone marrow transplantation after failure of conventional chemotherapy in adults with intermediate-Grade or high-Grade non-Hodgkin's lymphoma, " N. Engl. J. Med, 1987. 316(24): p. 1493-8; Gisselbrecht et al., "Salvage regimens with autologous transplantation for relapsed large B-cell lymphoma in the rituximab era, " J. Clin. Oncol., 2010. 28(27): p. 4184-90; Crump, et al., "Outcomes in refractory diffuse large B-cell lymphoma: results from the international SCHOLAR-1 study, " Blood, 2017. 130(16): p. 1800-1808). The most widely used high-dose therapy (HDT) regimen in the US involves the use of carmustine, etoposide, cytarabine, and melphalan and is commonly referred to as "BEAM" *(*Dahi, et al., "Strategies to improve outcomes of autologous hematopoietic cell transplant in lymphoma, " Bone Marrow Transplant, 2019. 54(7): p. 943-960). When compared to non-myeloablative chemotherapy plus radiotherapy, BEAM HDT followed by AHCT has demonstrated superior 5-year overall survival in patients with chemotherapy-sensitive relapsed lymphoma (Philip, et al., "Autologous bone marrow transplantation as compared with salvage chemotherapy in relapses of chemotherapy-sensitive non-Hodgkin's lymphoma, " N. Engl. J. Med., 1995. 333(23): p. 1540-5; Mills et al., "BEAM chemotherapy and autologous bone marrow transplantation for patients with relapsed or refractory non-Hodgkin's lymphoma, " J. Clin. Oncol., 1995. 13(3): p. 588-95). BEAM, together with BEAM-like alternatives, such as BeEAM (bendamustine replacing carmustine) represent the majority of modern day HDT for lymphoma (Vose et al., "Phase I trial of iodine-131 tositumomab with high-dose chemotherapy and autologous stem-cell transplantation for relapsed non-Hodgkin's lymphoma," J. Clin. Oncol., 2005, 23(3): p. 461-7; Friedberg, "Relapsed/refractory diffuse large B-cell lymphoma, " Hematology Am. Soc. Hematol. Educ. Program, 2011. 2011: p. 498-505; Chen et al., "Impact of conditioning regimen on outcomes for patients with lymphoma undergoing high-dose therapy with autologous hematopoietic cell transplantation, " Biol. Blood Marrow Transplant, 2015. 21(6): p. 1046-1053).

HDT-AHCT, although efficacious and potentially curative, is associated with severe regimen-related toxicities (SRRT) that increase patient morbidity and may lead to increase in mortality risk (Olivieri et al., "A Comparison of the Conditioning Regimens BEAM and FEAM for Autologous Hematopoietic Stem Cell Transplantation in Lymphoma:An Observational Study on 1038 Patients From Fondazione Italiana Linfomi. " Biol. Blood Marrow Transplant, 2018. 24(9): p. 1814-1822). SRRT is believed to occur primarily as a result of HDT off-target cytotoxicity involving multiple organs - predominantly organs with the highest cell turnover, with older patients and patients with co-morbid conditions exhibiting the highest SRRT incidence, severity, and mortality (Majhail et al., "Indications, for Autologous and Allogeneic Hematopoietic Cell Transplantation: Guidelines from the American Society for Blood and Marrow Transplantation, " Biol. Blood Marrow Transplant, 2015. 21(11): p. 1863-1869; Dahi et al., "Favorable outcomes in elderly patients undergoing high-dose therapy and autologous stem cell transplantation for non-Hodgkin lymphoma, " Biol. Blood Marrow Transplant, 2014. 20(12): p. 2004-9; Anderson et al., "Symptom burden in patients undergoing autologous stem-cell transplantation, "Bone Marrow Transplant, 2007. 39(12): p. 759-66).

Improvements in supportive care and transplantation practices over the last several decades have led to steady increases in the number of annual HDT-AHCTs performed. These improvements include the use of hematopoietic growth factors, infection prophylaxis and anti-emetics for chemotherapy induced nausea and vomiting (CINV). However, clinical signs and symptoms reflecting diffuse and severe mucositis of the oral-gastrointestinal tract, including severe nausea, vomiting and diarrhea that occur 3 to 5 days post-transplant, have not changed over the years. Similarly, neutropenic fever and infections continue to be frequent occurrences.

Grade ≥3 oral mucositis and nausea have been identified as two of the most severe adverse effects experienced with HDT-AHCT (Stiff, "Mucositis associated with stem cell transplantation: current status and innovative approaches to management, " Bone Marrow Transplant, 2001. 27 Suppl 2: p. S3-S11). In addition, many patients, because of the risk of severe toxicities or co-morbidities, are deemed transplant ineligible, and thus are deprived from a potentially curative therapy.

Patients undergoing HDT-AHCT for systemic lymphoma frequently experience SRRT. Most published studies indicate that Grade ≥3 AEs occur in up to 100% of patients [10, 24]. For patients treated with BEAM or similar conditioning regimens, the off-target cytotoxic damage to the gastrointestinal tract mucosa manifests as oral/GI SRRT (oral mucositis, nausea, vomiting and diarrhea) representing some of the most common and persistent severe and disabling symptoms. Published clinical studies indicate that rates of all-Grade oral mucositis in subjects undergoing BEAM conditioning can be as high as 61-84% with Grade ≥3 oral mucositis as high as 30% [10, 25]. This rate is increased in older patients as indicated in the study by *Dahi, et al.* where among subjects with median/range age 72 (70-77) years, the rate of patient-controlled analgesia use for Grade ≥3 oral mucositis was 40% [26]. Diarrhea is a very common adverse event after cytotoxic conditioning therapy with BEAM, with Grade ≥3 occurring in approximately 20% of patients, while Grade ≥3 nausea and vomiting occur in up to 20% of patients [10, 24].

In a large retrospective study of 1038 patients undergoing AHCT for lymphomas who received either BEAM or FEAM (fotemustine replacing carmustine) conditioning regimens, the median age of patients was 53 (range 16 to 79) and among 607 patients treated with BEAM, the rate of Grade ≥3 events of oral mucositis, nausea/vomiting, and diarrhea were 31%, 12% and 21% respectively. Olivieri, J., et al., A Comparison of the Conditioning Regimens BEAM and FEAM for Autologous Hematopoietic Stem Cell Transplantation in Lymphoma: An Observational Study on 1038 Patients From Fondazione Italiana Linfomi. Biol Blood Marrow Transplant, 2018. 24(9): p. 1814-1822*.* These findings were corroborated by a retrospective chart review study evaluating the rates of oral/GI SRRT in 143 patients treated in 2019-2020 with BEAM (96%) and having a median age of 58 (range 20,77). In this study, the rates of Grade ≥3 oral mucositis, nausea, vomiting and diarrhea were 10%, 20%, 2%, 26%, respectively, for a cumulative incidence of 41%. The rate of oral/GI SRRT was 25% in patients aged <40 and 46% in patients aged ≥40.

Importantly, the toxicities reported in both of these retrospective studies occurred despite the use of standard of care supportive care measures, which have not meaningfully improved in the last decade.

Hence there is a significant and unmet medical need for interventions that can enable patients to better tolerate HDT.

The present invention addresses this need. In particular, and as described in the below Examples, it has now been found that administration of allogeneic human umbilical cord endothelial cells (HUVECs) engineered to express the adenoviral E4ORF1 protein (i.e. "E4ORF1+ HUVECs" or "AB-205" cells) to human subjects with Hodgkin's or non-Hodgkin's lymphoma - as an adjunct to high-dose therapy (i.e. myeloablative chemotherapy) and autologous stem cell transplantation (HDT-ASCT) - is not only safe but is also effective in mitigating severe regimen-related toxicities.

Further details of the findings of this Phase 1 clinical trial are provided in Example 2 below. Details of various other protocols and studies are provided in Examples 1 and 3-5.

### Example 1

### Preparation of E4ORF1+ HUVECs Cells

Human umbilical vein endothelial cells (HUVECs) were isolated from the umbilical veins of allogeneic donor human umbilical cords by enzymatic digestion with collagenase. The umbilical veins were flushed with Hank's Balanced Salt Solution (HBSS) and then filled with a solution of 0.2% (w/v) collagenase and clamped at both ends. The cords were incubated at 20-25 °C for 30 minutes to allow for dissociation of endothelial cells from the umbilical vein. After 30 minutes, the detached cells were flushed out of the vein and transferred to a conical tube. The umbilical vein was washed again with HBSS and the wash volume added to the same conical tube. The tube was centrifuged, and the supernatant aspirated followed by resuspension of the pellet in 12 mL of an endothelial cell growth medium consisting of M199 base medium supplemented with 10% fetal bovine serum, 20 ng/mL FGF-2, 10 U/mL heparin, 40 µg/mL Gentamicin, 10 mM HEPES and 1X Glutamax. Cells were then transferred to endothelial cell growth medium in a T-75 flask and incubated at 37 °C, 5% CO2 for 2 days.

After 2 days in culture, HUVECs were transduced with a retroviral (MMLV) vector containing an E4ORF1 expression cassette (encoding SEQ ID NO.1) at a multiplicity of infection (MOI) of about 0.3-0.5. Four days after transduction, the endothelial cell growth medium was replaced with a serum-free selection medium containing gentamicin and the cells were cultured in the selection medium for five days - to select for the E4ORF1+ transduced cells. Cells were subsequently re-fed with endothelial cell growth medium and expanded in culture using closed-system bioreactors. Cryopreservation using a controlled-rate freezing program was employed as needed in order to bank the cells and/or hold the cells until needed. After cryopreservation cells were expanded again using closed-system bioreactors to generate sufficient cells for administration to patients. Assessment of cells produced by this approach showed that the mean viral copy number (the average number of copies of virus integrated into each cell in the population overall) was between approximately 0.88 and approximately 1.4. This is an average of the population as a whole and indicates that the composition can comprise both E4ORF1+ cells and E4ORF1- cells (endothelial cells that express E4ORF1 and those that do not). It further indicates that the population can comprise endothelial cells containing more than one copy of the E4ORF1 transgene.

### Example 2

### A Phase 1, Open Label, Non-randomized, Multi-Center Trial of AB-205 in Adults with Lymphoma Undergoing High-Dose Therapy and Autologous Stem Cell Transplantation

A Phase 1 clinical trial (clinicaltrials.gov identifier NCT03925935) was performed to assess the safety of administering allogeneic a therapeutic composition containing E4PRF1+ HUVECs (AB-205) to adult subjects with Hodgkin's or non-Hodgkin's lymphoma as an adjunct to high-dose chemotherapy (with or without radiation) and autologous stem cell transplantation (HDT-ASCT). E4ORF1+ HUVECs were prepared as described in Example 1.

This Example consists of three sub-sections - Example 2A, 2B and 2C. All relate to the same Phase 1 clinical trial. Example 2A provides data from an earlier stage in the trial where patients were treated in 3 cohorts (each receiving a single administration of E4ORF1+ HUVECs). Example 2B provides data from a later stage in the trial (with additional patients and longer follow-up) where patients were treated in 5 cohorts (cohorts 1-3 receiving a single administration of E4ORF1+ HUVECs and cohorts 4-5 receiving two administrations of E4ORF1+ HUVECs) and where additional data analysis was performed. Example 2C describes analysis of the provides

### Example 2A

### Safety & Efficacy of AB-205 at Multiple Doses with Single Administration Regimen

Adult subjects with Hodgkin's or non-Hodgkin's lymphoma who were in a chemo-sensitive remission undergoing high-dose chemotherapy, with or without radiation, and autologous stem cell transplantation (HDT-ASCT), were recruited.

Studies were performed to assess the safety of escalating single dose infusions of E4ORF1 HUVECs administered after HDT-ASCT by measuring the incidence of: (a) severe infusion-related toxicities within 24 hours of infusion, (b) all adverse events of Grade 3 of above based on the National Cancer Institute's common terminology criteria for adverse event version 5 ("NCI CTCAE v. 5.0") from the time of ASCT admission through day +100, and (c) mucosal toxicities including oropharyngeal mucositis, nausea, vomiting, and/or diarrhea, of Grade 3 and above.

Studies were also performed to assess the time to neutrophil engraftment (defined as the first of three consecutive days after ASCT of absolute neutrophil count (ANC)) ≥ 500/µL), the time to platelet engraftment (defined as the first of seven consecutive days after ASCT of platelet count ≥ 20,000/µL without transfusion support), lymphoid (T, B and NK cell) recovery at days +14, day +28, and day+100 post-ASCT, progression-free survival at days +100, +180, and +1 year post-ASCT, non-relapse mortality at +1 year post-ASCT, and overall survival at 1 year post-ASCT.

Enrolled subjects underwent their planned HDT-ASCT treatment for either Hodgkin or non-Hodgkin lymphoma using wither a BEAM, BeEAM, CBV, or TBC myeloablative conditioning regimen, as determined by the treating oncologist.

Local body radiation therapy was also permitted - as determined by the treating oncologist.

Autologous stem cell infusion was administered to the patients using standard protocols. The day on which the stem cell infusion was administered is designated as "day 0." After confirming that no acute reaction to the stem cell infusion occurred, a therapeutic composition consisting of E4ORF1+ HUVECs at a concentration of about 5.0 x 10⁶ cells per ml in a physiological saline solution (comprising dextran40 and HSA) was administered to the patients by intravenous infusion. The infusion of E4ORF1+ HUVECs was commenced 4 hours after completion of the autologous stem cell infusion and was completed within 2 hours. Different doses of the E4ORF1+ HUVECs were administered to each of three separate patient cohorts, as follows:
- Cohort 1 - 5 x 10⁶ cells per kilogram subject weight - day 0.
- Cohort 2 - 10 x 10⁶ cells per kilogram subject weight - day 0.
- Cohort 3 - 20 x 10⁶ cells per kilogram subject weight - day 0.

The below data is from 25 subjects treated in cohorts 1-3, 23 of whom were followed-up for at least 30 days (Example 2B includes data from longer patient follow-ups). The median age of the patients was 56 (range 22-69) with 12 subjects over the age of 55. The numbers of patients treated in each cohort was as follows:

**Table 2 - No. of Patients Treated in Cohorts 1-3**

| Cohort | E4ORF1+ HUVEC Cell Dose (cells/kg) | No. of Patients Treated |
|---|---|---|
| 1 | 5 x 10⁶ | 6 |
| 2 | 10 x 10⁶ | 10 |
| 3 | 20 x 10⁶ | 9 |
| Total No. Treated | | 25 |

Out of the 23 subjects followed up for at least 30 days, 16 subjects (70%) had lymphoma without CNS involvement and 7 subjects (30%) had CNS lymphoma. The subjects with CNS lymphoma involvement were all were treated with the CNS-penetrating regimen TBC, whether they had primary (n=6) or secondary (n=1) CNS involvement. (CNS-penetrating regimens, such as TBC, are known to have extremely high incidence and long duration of severe regimen-related multi-organ toxicities). The remainder of the subjects underwent HDT-ASCT with non-CNS penetrating conditioning regimens: standard BEAM, BeEAM or CBV conditioning (BEAM-like) - with or without rituximab. The characteristics of this group of patients are shown in Table 3, below.

**Table 3 - Subjects treated with Non-CNS-Penetrating Chemotherapy**

| Cell Dose (cells/kg) | Conditioning Regimen | Diagnosis | Age | Sex F/M |
|---|---|---|---|---|
| 5x10⁶ | BEAM | HL Nodular Sclerosis | 57 | F |
| 5x10⁶ | BEAM | Follicular NHL | 48 | M |
| 5x10⁶ | BeEAM | DLBCL | 68 | F |
| 5x10⁶ | BEAM | HL: Mixed Cellularity | 45 | M |
| 5x10⁶ | BEAM | DLBCL | 62 | M |
| 10x10⁶ | CBV | HL: Mixed Cellularity | 52 | F |
| 10x10⁶ | BEAM | Mantle Cell NHL | 41 | M |
| 10x10⁶ | BEAM | PTCL TFH | 54 | M |
| 10x10⁶ | BEAM | HL: classic | 38 | F |
| 10x10⁶ | BEAM | HL: classic | 49 | F |
| 10x10⁶ | BEAM | TCL: LC | 45 | M |
| 20x10⁶ | BEAM | Follicular NHL and DLBCL | 53 | M |
| 20x10⁶ | BEAM | HL: Nodular Sclerosis | 68 | M |
| 20x10⁶ | BEAM | Follicular NHL | 48 | F |
| 20x10⁶ | BEAM | DLBCL | 61 | M |
| 20x10⁶ | BEAM | DLBCL | 59 | F |

The abbreviations used in Table 3 are as follows: "HL" is Hodgkin's lymphoma; "NHL" is non-Hodgkin's lymphoma; "DLBCL" is diffuse large B cell lymphoma; "PTCL TFH" is peripheral T-cell lymphoma with Tfh phenotype; "TCL" is T cell lymphoma.

At the time of the analysis presented in Example 2A, all subjects were alive and without disease progression.

As described above, the NCI CTCAE system (version 5.0) was used to Grade adverse events (AEs). Adverse events (of any Grade ≥ 1) were reported in 100% of subjects, typical of subjects undergoing HDT-ASCT. Consistent with published data, subjects treated with CNS-penetrating HDT regimen exhibited greater incidence and duration of hematologic and organ toxicities compared to subjects treated with HDT regimens employed for non-CNS lymphoma (relapsed-refractory ("R/R") lymphoma). Specifically, 7 subjects conditioned with TBC had 60 CTCAE Grade 3-4 AEs including 30 Grade 3-4 non-hematologic toxicities including 7 episodes of febrile neutropenia in 5 subjects. Overall, there were 41 Grade 3-4 AEs. Number of G3-4 AEs is 17 (5 subjects), 15 (5/6 subjects), 9 (4/5 subjects) for cohort 1, 2, 3 respectively. As expected for HDT-ASCT, the overwhelming majority of Grade 3-4 AEs were hematologic.

Given the relatively high incidence of certain AEs involving the gastrointestinal tract and febrile neutropenia these events were assessed as indicators of organ toxicity. Specifically, the incidence of severe (Grade ≥3) gastrointestinal mucositis, Nausea/Vomiting/Diarrhea (N/V/D), and febrile neutropenia were used as indicators of the impact of administration of E4ORF1+ HUVECs on overall organ toxicity. Other supportive observations included, but were not limited to, the incidence of Grade 3+ nephritis, pulmonitis, CNS inflammation, hepatic inflammation and sepsis. Table 4 below summarizes some of the observed AE data.

**Table 4 - Adverse Events / Toxicity by E4ORF1+ HUVEC Cell Dose**

| Adverse Event (CTCAE Terminology, Grade ≥3) | 5x10⁶ cells/kg | 10x10⁶ cells/kg | 20x10⁶ cells/kg |
|---|---|---|---|
| Oral Mucositis, Nausea, Vomiting & Diarrhea | 0% | 0% | 0% |
| Febrile Neutropenia | 100% | 80% | 20% |

Importantly, no patient undergoing HDT-HCT with BEAM conditioning reported an incident of oral mucositis of Grade 3 or higher, suggesting that administration of E4PRF1+ HUVECs mitigated oral mucositis at all doses tested. The published rate of oral mucositis of at least Grade 3 in patients is ~ 20%.

For combined gastro-intestinal adverse events (oral mucositis, oral pain, nausea, vomiting, and diarrhea) there were no Grade 3-4 AEs - again suggesting that administration of E4ORF1+ HUVECs mitigated these AEs at all doses tested. There were 15 Grade 2 AEs in 7 (43%) subjects. There were only 5 Grade 3-4 non-hematologic end-organ AEs in 3 (19%) subjects, including Grade 3 epistaxis, rhinovirus infection, hypokalemia, hyperkalemia, and Grade 4 supraventricular tachycardia.

Significantly, the incidence of febrile neutropenia decreased from 100% (5/5) in cohort 1, to 80% (5/6) in Cohort 2 and 20% (1/5) subjects in Cohort 3 - demonstrating that administration of E4ORF1+ HUVECs decreases febrile neutropenia in a dose-dependent manner.

The following observations were also made. Only two patients not undergoing TBC-containing regimens reported severe (Grade 3 or higher) side effects. These included one event each of epistaxis (nose-bleed) which resolved within two days, infectious enterocolitis which resolved within three days, and sepsis which resolved within one day. There were no other reports of more than moderate side effects in any patient not receiving TBC regimen. No side effects of depression, delusion, delirium, euphoria, hallucination, change (increase or decrease) in libido, mania, personality change, or suicidal ideation were reported. One patient reported mild confusion. Only one patient reported any atrial dysfunction; that patient received the TBC regimen. No patient reported cardiac arrest, conduction disorder, heart failure, disease of the cardiac valves, myocardial infarction, myocarditis, pericardial effusion, pericarditis, fibrillation, or ventricular tachycardia. One patient reported mild palpitations (Grade 1) that resolved within one day. No patient reported ear pain, loss of hearing, tinnitus, or vertigo. The only dizziness reported was mild (CTCAE v5; Grade 1). No endocrine disorders were reported. The only ocular side effect reported in any patient was blurred vision. No patient reported development of cataracts, corneal ulceration, dry eye, or any other ocular disorder. No patient reported anal mucositis, development of anal fistula, colitis, and several other gastrointestinal side effects. Only one patient reported severe abdominal pain. This was a patient receiving the TBC regimen. Other than as noted above, other gastro-intestinal side effects reported were mild or moderate and most were of short duration.

In addition to routine tests performed as part of standard care, all subjects had blood tests performed at baseline, day 2, 7, 14, 21, 28, 56, and day 100. No patient exhibited a clinically significant change (elevation or decrease) in blood urea nitrogen, serum creatinine, chloride, phosphate, sodium, bicarbonate, aspartate amino transferase, alanine aminotransferase, alkaline phosphatase, albumin, total protein, total bilirubin, direct bilirubin, or lactate dehydrogenase at any of these time points. Only two patients, both receiving TBC regimen, exhibited a clinically significant change in calcium; for one of these patients the change was limited to a single test day (day 2). Only two patients, both receiving TBC regimen, exhibited a clinically significant change in magnesium; for both of these patients the change was limited to a single test day. Only one patient, receiving the BEAM regimen, exhibited a clinically significant change in glucose; this was evident at baseline (prior to treatment) and at day 0 and day 2 but not reported thereafter.

Only two patients reported edema; both received the TBC regimen; one was reported as mild (Grade 1), the other moderate (Grade 2). The majority of patients reporting fatigue reported only Grade 1. Only one patient reported generalized muscle weakness. Only three patients reported issues associated with liver dysfunction (elevated bilirubin, elevated transferase enzymes); all three were considered mild (Grade 1). No urinary tract issues were reported other than a single urinary tract infection that was deemed moderate (Grade 2). Three patients reported peripheral sensory neuropathy two of whom had received the TBC regimen-one was deemed Grade 1 and the other Grade 2 (moderate). Only one patient reported dysmenorrhea, this patient received TBC; the dysmenorrhea was deemed mild (Grade 1).

The median time to platelet engraftment was 9 (range 0-14) while the median date to neutrophil engraftment was 10 (range 8-13) in 13 subjects with verified engraftment cell counts. Platelet engraftment preceded neutrophil engraftment in 6 (46%) subjects while all 13 (100%) subjects engrafted platelets either prior to or within 1 day of neutrophil engraftment.

Some of the above data is represented in Figs. 1- 4.

### Example 2B

### Expanded Study of Safety & Efficacy of AB-205 Treatment and Multiple Dodes Delivered Using Either in a Single or Double Administration Regimen

This Example includes data from 35 subjects with systemic lymphoma (without CNS involvement) who have been treated with a therapeutic composition comprising E4ORF1+ HUVECs (i.e. AB-205) and followed up for at least 100 days. This Example includes dose escalation data obtained following administration of from 5×10⁶ to 20×10⁶ E4ORF1+ HUVEC cells/kg administered after AHCT on Day 0, with or without an additional 10×10⁶ cells/kg administered on Day 2. Subjects received the following HDT regimens: BEAM (86%), BeEAM (11%) or CBV (4%). Oral/GI SRRT were defined as NCI-CTCAE (v5.0) Grade ≥3 oral mucositis, nausea, vomiting or diarrhea occurring from the start of HDT through Day 28 post AHCT.

In brief, no clinically meaningful adverse events considered directly related to AB-205 treatment occurred in the study. Furthermore, the data from the study showed that administration of E4ORF1+ HUVECs resulted in low rates of oral/GI SRRTs overall (with an incidence rate of 3/35 or 9%) and a dose-responsive reduction in oral/GI toxicities and febrile neutropenia. No carditis, pneumonitis, nephritis or hepatitis regimen related toxicities reported, and 68% of subjects achieved platelet engraftment prior to or within one day of neutrophil engraftment.

The primary objective of this clinical study was to evaluate the safety of AB-205 as assessed by the number of dose-limiting toxicities (DLT) in the dose escalating cohorts of AB-205.

Key eligibility criteria for this study included the following: Age ≥ 18 years old; Diagnosis of HL or NHL; Candidates for HDT-AHCT with either BEAM, BeEAM, CBV or TBC myeloablative conditioning regimens (BEAM, BeEAM or CBV for systemic lymphoma patients or TBC for CNS lymphoma patients). Eligible subjects were enrolled into sequential dose-escalating cohorts, as follows:
Cohort 1: 5×10⁶ cells/kg on Day 0
Cohort 2: 10×10⁶ cells/kg on Day 0
Cohort 3: 20×10⁶ cells/kg on Day 0
Cohort 4: 10×10⁶ cells/kg on Day 0 and 10×10⁶ cells/kg on Day 2
Cohort 5: 20×10⁶ cells/kg on Day 0 and 10×10⁶ cells/kg on Day 2

AB-205 was administered on Day 0, after AHCT. Subjects were permitted to receive standard-of-care (SOC) prophylaxis and supportive care, including: growth factors, oral cryotherapy (ice chips), infection prophylaxis / treatment, anti-emetics, blood product transfusions, nutritional support, and other appropriate care.

42 subjects for whom follow-up was performed for at least 100-days post-AHCT were enrolled into cohorts 1 (n=6), 2 (n=10), 3 (n=10), 4 (n=10) and 5 (n=6). The demographic characteristics of the subjects were representative of those of the larger community of lymphoma patients that undergo HDT-AHCT, as shown in Table 5, below, with no meaningful differences across cohorts.

**Table 5: AB-205-001 Demographics and Baseline Characteristics**

| | **Systemic Lymphoma (N=35)** | | **CNS Lymphoma (N=7)** | |
|---|---|---|---|---|
| Age (years), median (Range) | 53 (20, 73) | | 59 (23, 69) | |
| Age ≥ 60 (%) | 31 | | 43 | |
| Male (%) | 51 | | 57 | |
| Diagnosis, n (%) | HL | 12 (34) | PCNSL | 5 (71) |
| | NHL | 23 (66) | SCHSL | 2 (29) |
| Prior lines of treatment, n (range) | 2 (1, 4) | | 1 (1, 3) | |
| Conditioning Regimen, n (%) | BEAM | 29 (83) | | |
| | BeEAM | 5 (14) | TBC | 7(100) |
| | CBV | 1 (3) | | |
| CD34+ Count ×10⁶/kg, median (range) | 5.2 (2.8, 19.6) | | 3.8 (2.0, 8.3) | |
| DLBCL = diffuse large B cell lymphoma, FL = follicular lymphoma; HL = Hodgkin lymphoma; MCL = mantle cell lymphoma; NHL = non-Hodgkin lymphoma; PCNSL = primary CNS lymphoma; SCNSL = secondary CNS lymphoma; TCL = T-cell lymphoma | | | | |

No maximum tolerated dose (MTD) was established. There was only one subject who met the definition of prospectively described dose-limiting toxicity (DLT) - exhibiting prolonged Grade 4 thrombocytopenia with a duration greater than 28 days. However, this event was deemed to be unrelated to the AB-205 treatment. No DLTs were observed in the subjects treated with BEAM-like conditioning. Importantly, there were no significant infusion related reactions.

Adverse events (AEs) of any Grade (CTCAE Grade ≥ 1) were reported in 100% of subjects, typical of subjects undergoing HDT-AHCT. The most frequently reported non-hematologic treatment-emergent AEs (TEAEs) amongst the subjects with systemic lymphoma (n=35) were diarrhea (71%), nausea (46%), stomatitis (46%) and fatigue (43%) (Table 6).

**Table 6 - All Adverse Events: Systemic Lymphoma**

| **Cohort No.** | **1** | **2** | **3** | **4** | **5** | **All** |
|---|---|---|---|---|---|---|
| No. of subjects | 5 | 6 | 8 | 10 | 6 | 35 |
| Any event* | 5 (100) | 6 (100) | 8 (100) | 10 (100) | 6 (100) | 35 (100) |
| Diarrhea | 3 (60) | 6 (100) | 3 (38) | 9 (90) | 4 (67) | 25 (71) |
| Nausea | 4 (80) | 3 (50) | 3 (38) | 4 (40) | 2 (33) | 16 (46) |
| Stomatitis | 2 (40) | 4 (67) | 4 (50) | 5 (50) | 1 (17) | 16 (46) |
| Fatigue | 2 (40) | 2 (33) | 6 (75) | 1 (10) | 4 (67) | 15 (43) |
| Decreased appetite | 3 (60) | 1 (17) | 2 (25) | 3 (30) | 1 (17) | 10 (29) |
| Vomiting | 3 (60) | 2 (33) | 1 (13) | 2 (20) | 1 (17) | 9 (26) |
| Abdominal pain | 0 | 3 (50) | 1 (13) | 4 (40) | 0 | 8 (23) |
| Hypocalcemia | 2 (40) | 1 (17) | 3 (38) | 1(10) | 1 (17) | 8 (23) |
| Hypokalemia | 2 (40) | 0 | 4 (50) | 1 (10) | 1 (17) | 8 (23) |
| Headache | 0 | 2 (33) | 4 (50) | 1 (10) | 0 | 7 (20) |
| Pyrexia | 1 (20) | 1 (17) | 2 (25) | 2 (20) | 1 (17) | 7 (20) |
| * Includes hematologic (%) | | | | | | |

The most commonly reported non-hematologic TEAEs (>30%) amongst the subjects with CNS involvement (n=7) were fatigue (57%), pyrexia (57%), stomatitis (57%), esophagitis (43%) and rash (43%). As expected for subjects undergoing HDT-AHCT, the majority of Grade ≥3 AEs were hematologic. Non-hematologic Grade ≥3 AEs reported for subjects with systemic lymphoma (n=35) are shown in Table 7.

**Table 7 - Grade ≥3 Non-Hematologic TEAEs: Systemic Lymphoma**

| **Cohort (no. of subjects)** | **1 (n=5)** | **2 (n=6)** | **3 (n=8)** | **4 (n=10)** | **5 (n=6)** | **All (n=35)** |
|---|---|---|---|---|---|---|
| Any event* | 5 (100) | 5 (83) | 8 (100) | 7 (70) | 5 (83) | 30 (86) |
| Hypokalemia | 1 (20) | 0 | 1 (13) | 1 (10) | 0 | 3 (9) |
| Stomatitis | 0 | 1 (17) | 0 | 0 | 1 (17) | 2 (6) |
| Colitis | 0 | 0 | 0 | 1 (10) | 0 | 1 (3) |
| Diarrhea | 0 | 0 | 0 | 0 | 1 (17) | 1 (3) |
| Esophagitis | 0 | 0 | 0 | 0 | 1 (17) | 1 (3) |
| C. difficile colitis | 0 | 0 | 1 (13) | 0 | 0 | 1 (3) |
| Sepsis | 0 | 0 | 1 (13) | 0 | 0 | 1 (3) |
| Septic shock | 0 | 0 | 0 | 0 | 1 (17) | 1 (3) |
| Angina pectoris | 0 | 0 | 1 (13) | 0 | 0 | 1 (3) |
| SVT | 0 | 1 (17) | 0 | 0 | 0 | 1 (3) |
| Hypocalcemia | 0 | 0 | 0 | 1 (10) | 0 | 1 (3) |
| Hypophosphatemia | 0 | 0 | 1 (13) | 0 | 0 | 1 (3) |
| Syncope | 0 | 0 | 0 | 0 | 1 (17) | 1 (3) |
| Depression | 0 | 0 | 1 (13) | 0 | 0 | 1 (3) |
| Epistaxis | 1 (20) | 0 | 0 | 0 | 0 | 1 (3) |
| * Includes hematologic | | | | | | |

Grade ≥3 non-hematologic TEAEs reported for subjects with CNS lymphoma (n=7) were esophagitis (n=3), stomatitis (n=3), rash (n=2) and n=1 for the atrial fibrillation, lung infection, herpes simplex infection, device related infection, anxiety, DVT and hypertension.

There were 25 SAEs reported in 17/42 (40%) subjects including 16 SAEs that occurred in 11/35 (31%) subjects with systemic lymphoma, and 9/25 SAEs that occurred in 6/7 (86%) subjects with CNS lymphoma. Lung infection / pneumonia (n=4), febrile neutropenia (n=3) and fever (n=2) were the only SAEs that occurred in more than one subject.

TEAEs considered related to AB-205 by investigators were rare. Amongst subjects with systemic lymphoma, the TEAEs considered related to AB-205 were (all n=1) diarrhea (Grade 1, Cohort 2), tooth pain (Grade 1, Cohort 2), rigors (Grade 3, Cohort 4). Three Grade 3 TEAEs were considered related to AB-205 in subjects with CNS lymphoma: rash, febrile neutropenia and thromboembolic event. The thromboembolic event was a SAE of acute deep vein thrombosis that occurred on Day 28 after AB-205 treatment and was deemed unrelated to treatment.

Overall, no concerning safety signals attributed to AB-205 infusion were observed in this Phase 1 trial. There were no clinically significant infusion related toxicities, and no MTD was reached.

Oral/GI SRRTs were defined as NCI CTCAE version 5.0 Grade ≥3 events of oral mucositis, nausea, vomiting or diarrhea from the start of HDT (i.e., before dosing with AB 205 on Day 0) until Day 28 post AHCT. The following data describe the systemic lymphoma subjects without CNS involvement (n=35) in the AB-205-001 study. Treatment with AB-205 achieved low rates of oral/GI SRRT overall with an incidence rate of 9% (3/35 subjects) (Table 8).

**Table 8 - Oral/GI SRRT in Subjects with Systemic Lymphoma**

| **Grade ≥ 3** | **Cohort 1 (n=5)** | **Cohort 2 (n=6)** | **Cohort 3 (n=8)** | **Cohort 4 (n=10)** | **Cohort 5 (n=6)** | **Overall (n=35)** |
|---|---|---|---|---|---|---|
| Oral/GI SRRT, n (%) | 0 | 1 (17) | 0 | 0 | 2 (33) | 3 (9) |
| 95% CI | (0, 52) | (0, 64) | (0, 37) | (0, 31) | (4, 78) | (2, 23) |
| | | | | | | |
| Oral Mucositis | 0 | 1 (17) | 0 | 0 | 1 (17) | 2 (6) |
| Diarrhea | 0 | 0 | 0 | 0 | 1 (17) | 1 (3) |
| Nausea / Vomiting | 0 | 0 | 0 | 0 | 0 | 0 |

The 3 subjects who experienced oral/GI SRRT had risk factors for GI toxicities. When the Phase 3 eligibility criteria (age ≥ 40 years old) are applied to these AB-205 study results, no systemic lymphoma subjects experienced oral/GI SRRT after dosing with AB-205.

Across all cohorts, the median (IQR) time to neutrophil and platelet engraftment were 10 days (9, 11) and 11 days (10, 13) respectively.

Two dosing schedules were evaluated in this Phase 1 study - a first dosing schedule consisting of a single administration on Day 0, and a second dosing schedule consisting of two administrations - one on Day 0 and one on Day 2. The additional dose on Day 2 showed some but limited incremental efficacy compared to dosing on Day 0 alone.

As all dose levels of AB-205 substantially reduced the incidence of oral/GI SRRT, further analyses were conducted evaluating all Grade oral/GI RRT (i.e., mild, moderate, and severe oral/GI toxicities) and incidence and duration of febrile neutropenia (FN). In view of relatively small patient numbers at each dose level, cohorts were grouped according to the most impactful dose of AB-205, i.e., the Day 0 dose, as follows:
Group 1: <20×10⁶ cells/kg on D0, i.e., Cohorts 1, 2 and 4 (n=21)
Group 2: 20×10⁶ cells/kg on D0, i.e., Cohorts 3 and 5 (n=14)

The occurrence of all Grades of oral/GI events and Grade ≥ 3 febrile neutropenia (FN) in Group 1 and Group 2 are presented in Table 9.

**Table 9: All Grade Oral/GI RRT and Grade ≥ 3 FN**

| | **Group 1** | **Group 2** |
|---|---|---|
| Oral/GI RRT, n (%) | 21 (100) | 13 (93) |
| Oral Mucositis | 12 (57) | 8 (57) |
| Diarrhea | 17 (81) | 7 (50) |
| Nausea / Vomiting | 17 (81) | 9 (64) |
| Febrile Neutropenia (Grade ≥3) | 15 (71) | 7 (50) |

Considering all Grade oral/GI events, there was a dose response in the components of the composite endpoint when comparing subjects who received less than 20×10⁶ cells/kg on Day 0 with those who received 20×10⁶ cells/kg on Day 0.

Treatment with AB-205 further resulted in a dose dependent reduction in the incidence of febrile neutropenia (FN). Moreover, amongst subjects with FN the median (range) duration of febrile neutropenia was only 2 (1, 3) days for subjects receiving 20×10⁶ cells/kg on Day 0 versus 4 (1, 9) for subjects receiving less than 20×10⁶ cells/kg on Day 0.

**Table 10 - Effect of AB-205 on Recovery of Neutrophils and Platelets**

| | **AB-205** | **Control** | **Delta** | **P-value** |
|---|---|---|---|---|
| Number (no.) | 35 | 143 | | |
| Neutrophil Engraftment (mean, days) | 10.17 | 12.23 | -2.06 | <0.0001 |
| Platelet Enggraftment (mean, days) | 12.63 | 17.57 | -4.94 | 0.0013 |
| Platelet within 1 day of Neutrophil (%) | 60% | 31% | 29% | 0.0025 |

**Table 11 - Effect of AB-205 on Duration of SRRT and Hospitalization**

| **Systemic Lymphoma Undergoing HDT-AHCT** | **Control Cohort (N=143)** | **AB-205 (N=35)** |
|---|---|---|
| N (%) with Oral/GI SRRT | 59 (41%) | 3 (9%) |
| Median duration of Oral/GI SRRT (range) | 12 days (4, 38) | 2 days (1, 6) |
| Median Length of Stay (range) | 15 days (9, 42) | 12 days (0, 45) |

Some of the above data, as well as additional data from this clinical trial, is represented in **Figs. 6-9****.**

In conclusion, the data from this Phase 1 clinical trial demonstrated that, at all doses and dosing schedules tested, AB-205 is safe for administration to patients with various different types of lymphoma undergoing HDT-AHCT, with no MTD being reached, no infusion-related reactions noted, and no reports of cardiac, renal or hepatic RRTs. Furthermore, and importantly, the data from this Phase 1 (Phase 1b/2) clinical trial provided evidence of efficacy of AB-205 treatment for mitigating the side-effects of HDT. Thus, lower than normal rates of oral/GI SRRT were observed across all AB-205 doses tested overall (9% (95% CI: 2, 23), with a dose-responsive effect seen for oral/GI RRTs and febrile neutropenia (FN). Moreover, the data demonstrated that AB-205 treated patients had early neutrophil and platelet recovery.

### Example 2C

### Safety and Efficacy of AB-205 Treatment Analyzed by Patient Age

Data from the Phase 1 clinical studies described above was analyzed by age of the subjects treated. The data analyzed was obtained from subjects with chemo-sensitive lymphoma treated with intravenous E4ORF1+ HUVECs at a dose of 20x10⁶ cells/kg - either as single dose administered on Day 0 after AHCT or as a divided dose administered on Day 0 and Day 2 after AHCT. Supportive care therapies were also administered, as described above. Oral/GI SRRTs were defined as NCI-CTCAEv5.0 Grade ≥3 oral mucositis, nausea, vomiting or diarrhea. Twenty-nine (29) subjects were treated, with a median follow up time of 271 days (range 179-566). A retrospective chart review (n=45) at a participating site served as a contemporary control. Maximum tolerated dose (MTD) was not reached. As of the cutoff date of this study, there were no reports of SRRT affecting the pulmonary, cardiac, renal or hepatic systems and no detrimental effect on PFS or OS was observed. There were also no deaths by day 100 after treatment. The control cohort reported an oral/GI SRRT rate of 16/25 (64%) in subjects ≥ 60 years old, while subjects <60 years old reported an oral/GI SRRT rate of 9/20 (45%). Thus, the rate of oral/GI SSRTs was much higher in subjects over the age of 60.

However, the E4ORF1+ HUVEC treatment effectively eliminated oral/GI SSRT in both the older and younger groups - no oral/GI SRRTs were reported for any of the subjects receiving the AB-205 treatment (0%, n=18), including all of the subjects ≥ 60 years in age (n=7). The median time to neutrophil engraftment (days), the median time to platelet engraftment, and the number of hospitalization days (from AHCT) were also lower in the treated groups as compared to the control cohorts - by approximately 2, 7 and 3 days, respectively. Thus, even though the older patients exhibited much higher rates of oral/GI SRRTs, E4ORF1+ HUVEC treatment was still highly effective - eliminating oral/GI SRRTs across age groups. Importantly, this data shows that treatment with E4ORF1+ HUVECs has significant potential to make HDT-AHCT substantially safer for patients of all ages - including older patients who may previously not have been considered candidates for HCT because of their high rates of SRRT. Data from this analysis is provided in Table 12, below.

**Table 12 - Treatment (E4ORF1+ HUVEC) vs. Control (Retrospective Chart Data) Analyzed by Patient Age**

| | **Age <60** | | **Age ≥60** | |
|---|---|---|---|---|
| | **AB-205** | **Control** | **AB-205** | **Control** |
| Number of subjects (N) | 11 | 20 | 7 | 25 |
| Age, Median (range), years | 47 (25, 59) | 40 (21, 59) | 66 (61, 73) | 66 (60, 73) |
| Male (%) | 55 | 65 | 57 | 80 |
| Diagnosis HL/NHL (%) | 27/73 | 55/40 | 29/71 | 8/92 |
| Lines of Therapy, median (range) | 2 (1, 4) | 2 (1, 3) | 2 (2, 2) | 2 (1,4) |
| HDT BEAM/BeEAM (%) | 91/9 | 95/5 | 86/14 | 96/4 |
| CD34+ cells x10⁶/kg, median (range) | 4.2 (2.8, 16.7) | 5.3 (3.2-7.9) | 6.3 (4.5, 8.4) | 3.2 (2.0-7.2) |
| Oral/GI SRRT, n (%) | 0 | 9 (45) | 0 | 16 (64) |
| Neutrophil engraftment, median, days | 10 | 12 | 10 | 13 |
| Platelet engraftment, median, days | 11 | 21 | 11 | 18 |
| Number of hospitalization days from AHCT, median, days | 13 | 16 | 12 | 16 |

### Example 3

### A Phase 3 Study of the Effects of AB-205 in Patients with Lymphoma Undergoing Autologous Hematopoietic Cell Transplantation

A Phase 3 clinical trial is performed to confirm the safety and efficacy of AB-205 demonsrated in the Phase 1 (Phase 1b/2) study described above in a larger patient population. Specifically, a double-blind, randomized, placebo controlled multi-center clinical trial is performed in adult subjects (≥ 40 years in age) with lymphoma (Hodgkin lymphoma (HL) or non-Hodgkin lymphoma (NHL)) who are in a chemo-sensitive remission and are candidates for HDT-AHCT with either a BEAM (carmustine, etoposide, cytarabine, melphalan) or BeEAM (bendamustine, etoposide, cytarabine, melphalan) conditioning regimen.

Eligible subjects are randomized in a 1:1 ratio into "treatment" (Arm A - AB-205 plus standard of care) and "control" (Arm B - placebo plus standard of care) groups. Approximately 140 subjects are treated with the investigational product (IP) - i.e., AB-205. Subjects in both Arm A and Arm B are treated with a HDT regimen - either BEAM or BeEAM. Subjects in Arm A receive AB-205 at a dose of 20×10⁶ cells/kg on day 0 (i.e., the day that the AHCT is performed) - approximately three hours (± 1 hour) after the AHCT is performed. A 20×10⁶ cells/kg dose of AB-205 was selected as this dose was well tolerated and exhibited maximal efficacy for SRRT reduction in the Phase 1 study. (An additional dose of AB-205 administered on day 2 in the Phase 1 study afforded no meaningful incremental efficacy). Subjects in Arm B are treated the same as those in Arm A but receive a placebo (vehicle without cells) instead of AB-205. Additional details of the protocols for Arm A and Arm B are provided in Table 13, below.

**Table 13- Protocol for Treatment and Control Arms of Phase 3 Clinical Trial**

| | **Arm A** | **Arm B** |
|---|---|---|
| Intervention Name | AB-205 | Placebo |
| Description | E4ORF1+ HUVECs thawed and diluted in a final diluent concentration of 7.9% | Solution consisting of: |
| | | 4.25% HSA |
| | | 8.3% Dextran40 in normal saline. |
| | Dextran40 and 4.5% HSA with a residual of 0.25% of DMSO in normal saline. | |
| Unit Dose Strength(s) | 5×10⁶ E4ORF1+ HUVEC cells / mL | NA |
| Dosage Level(s) | 20×10⁶ E4ORF1+ HUVEC cells / kg body weight. | An analogous volume i.e. (20×Weight/5) mL |
| | Volume to be administered is calculated as (20×Weight/5) mL | |
| Route of Administration | IV infusion | IV infusion |
| Timing of Administration | 3 hours (± 1 hour) after completion of AHCT | 3 hours (± 1 hour) after completion of AHCT |
| Rate of Administration | 6 mL/min i.e., 30×10⁶ cells / min | 6 mL/min |

Subjects undergo evaluations of primary and secondary endpoints as described in Table 12. Assessments of oral/GI SRRTs are made daily from the start of HDT through Day 21 post AHC. Long-term follow up for other SRRTs, disease status, and survival is performed through to 1 year post AHCT.

**Table 14 - Primary and Secondary Objectives & Endpoints of Phase 3 Trial**

| **Objectives** | **Endpoints** | |
|---|---|---|
| Primary | | |
| • To demonstrate a reduction in the incidence and severity of regimen related toxicities with AB-205 plus standard of care (SoC) versus placebo plus SoC in adults with lymphoma undergoing HDT-AHCT | • The primary endpoint is complete response, defined as the absence of oral/GI severe regimen related toxicities (oral/GI SRRT). Oral/GI SRRT are defined as NCI CTCAE v. 5.0 Grade (G) ≥ 3: Oral mucositis (OM), nausea (N), vomiting (V), or diarrhea* (D) from the time of HDT administration through Day 21 after AHCT | |

| Secondary | | |
|---|---|---|
| • To assess the efficacy of AB-205 versus placebo in adults with lymphoma undergoing HDT AHCT | Key Secondary Endpoints | |
| | AHCT occurs on Day 0 | |
| | • Duration of oral/GI SRRT | |
| | • Symptom burden per MDASI (MD Anderson Symptom Inventory) | |
| • To assess the safety of AB-205 in adults with lymphoma undergoing HDT-AHCT | | |
| | • Duration of febrile neutropenia (FN) | |
| | • Time to hematopoietic engraftment (i.e., neutrophil *and* platelet) | |
| | Other Secondary Endpoints | |
| | • Incidence of Grade ≥3 non-hematologic AEs | |
| | • The absence of non-GI solid-organ SRRT. Non-GI solid organ SRRT are defined as the following NCI CTCAE v. 5.0 Grade (G) ≥ 3 from the time of HDT administration through Day 100 after AHCT: | |
| | | ∘ Myocarditis, pericarditis or myocardial infarction; |
| | | ∘ Non-infectious pneumonitis or respiratory failure; |
| | | ∘ Acute kidney injury or nephrotic syndrome |
| | | ∘ Hepatic failure or sinusoidal obstructive syndrome |
| | • Time to platelet engraftment | |
| | • Time to neutrophil engraftment | |
| | • Number of red blood cell (RBC) transfusions and days to last RBC transfusion from AHCT | |
| | • Number of platelet transfusions and days to last platelet transfusion from AHCT | |
| | • All Grade oral/GI regimen related toxicities (RRT) | |
| | • Opiate usage defined as number of days with opiate usage through Day 28 | |
| | • Survival endpoints at Day 100 and Day 365 post AHCT: | |
| | | ∘ Non-relapse mortality (NRM) |
| | | ∘ Progression free survival (PFS) |
| | | ∘ Overall survival (OS) |
| | Safety | |
| | Safety assessments include all Grade AEs, severe AEs (SAE), physical examinations, vital sign assessments, clinical laboratory evaluations and ECGs. | |
| Health Economics and Outcomes Research | | |
| • Burden of illness | • Hospitalization days through Day 100 post AHCT, including number of readmissions and length of stay of each admission | |
| | • Number of outpatient visits through Day 100 | |
| | • Healthcare Resource Utilization per CPT codes through Day 100 | |
| | • Employment status at 6 and 12 months | |

The secondary endpoint of "time to hematopoietic engraftment" is defined as the later of time to neutrophil engraftment and time to platelet engraftment, where: time to neutrophil engraftment is defined as the number of days to the first of three consecutive days after AHCT with absolute neutrophil count (ANC) > 500/µL, and time to platelet engraftment is defined as the number of days to the first of seven consecutive days after AHCT of platelet count ≥ 20,000/µL without transfusion support. Daily hematology samples are drawn for analysis of these endpoints until engraftment has occurred.

### Example 4

### Supportive Therapy / Standard of Care

Standard of care supportive therapy may be provided to subjects treated with E4ORF1+ HUVECS if determined to be appropriate by a treating physician or if consistent with any institutional guidelines.

Standard of care supportive therapy can include therapy for: oral mucositis prophylaxis, chemotherapy induced nausea and vomiting (CINV) prophylaxis, bacterial infection prophylaxis, and anti-infective prophylaxis, and blood and platelet transfusion support. Treatment of any emergent toxicities can also be provided if needed.

CINV prophylaxis may involve administration of a corticosteroid (e.g., dexamethasone), a 5 HT3 antagonist (e.g, ondansetron), and/or an NK1 antagonist (e.g., aprepitant).

Anti-bacterial prophylaxis for gram negative bacterial infections may comprise administration of a quinolone (e.g. levofloxacin) e.g., from Day +1 or ANC < 1000 cells/µL until initiation of broad spectrum antibiotics or ANC > 1000/µL. For subjects with intolerance or allergy to quinolones, a third-generation cephalosporin such as ceftriaxone or cefpodoxime may be used (e.g., 200 mg orally twice a day on Day +1).

Hematopoietic support may comprise administration of G-CSF, e.g., at about 300 µg/day (for subjects weighing < 75kg) or about 480 µg/day (for subjects weighing ≥ 75kg), e.g., from day 5 post AHCT until neutrophil engraftment.

Platelet support may be provided as needed.

**Table 15 - Examples of Standard of Care Supportive Therapy Regimens**

| **Supportive Care** | | | | | | | | | |
|---|---|---|---|---|---|---|---|---|---|
| **CINV Prophylaxis** | | | | | | | | | |
| Ondansetron | - | 16 mg IV | 8 mg IV | 8 mg IV | 8 mg IV | 8 mg IV | 16 mg IV | 8 mg IV | - |
| Aprepitant | - | - | - | - | - | - | 150 mg IV | - | - |
| Dexamethaso ne | - | 12 mg IV | | | | | 12 mg IV | 8 mg IV | |

| **Oral Mucositis** | | | | | | | | | |
|---|---|---|---|---|---|---|---|---|---|
| Oral care regimen | All time | | | | | | | | |
| Cryotherapy | | - | - | - | - | - | 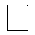 | - | - |

| **Anti-Bacterial Infection Prophylaxis** | | | | | | | | | |
|---|---|---|---|---|---|---|---|---|---|
| Quinolone | - | - | - | - | - | - | - | - | 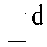 |

Levofloxacin may be administered at about 500 mg orally or intravenously daily. Ciprofloxacin may be administered at about 500 mg orally twice daily or about 400 mg intravenously every 12 hours from Day +1 or ANC < 1000 cells/µL until initiation of broad-spectrum antibiotics or ANC > 1000/µ. In subjects with intolerance to quinolone, a cephalosporin (such as ceftriaxone or cefpodoxime) may be administered at about 200 mg orally twice a day on Day +1.

### Example 5

### A Phase 1 Clinical Trial of E4ORF1+ HUVECs in Adults with Leukemia or Myelodyplastic Syndrome Undergoing High-Dose Therapy and Allogeneic Stem Cell Transplantation

In addition to the clinical studies described above, which involve administering E4ORF1+ HUVECs (AB205) cell to human subjects with Hodgkin's or non-Hodgkin's lymphoma as an adjunct to high-dose chemotherapy and autologous stem cell transplantation (HDT-ASCT), a separate clinical were performed to assess the safety of administering E4ORF1+ HUVECs to adult subjects with leukemia (e.g., acute myeloid leukemia (AML) or acute lymphoblastic leukemia (ALL)) or myelodysplastic syndrome as an adjunct to high-dose chemotherapy and allogeneic stem cell transplantation (HDT-AlloSCT) (clinicaltrials.gov identifier NCT03483324).

E4ORF1+ HUVECs were prepared as described in Example 1.

Adult subjects with leukemia or myelodysplastic syndrome and tandem (two donor) umbilical cord unit blood stem cell transplantation (tandem HDT-Allo-SCT), were recruited and enrolled in three cohorts in an open label, multi-center study. In the first two cohorts one of the cord blood units was expanded in culture in the presence of E4ORF1+ HUVEC cells. This expanded unit and the E4ORF1+ HUVEC cells were then infused into the patients together along with the second unit that was not expanded in culture. In a third cohort both units are infused without culture expansion with a concomitant infusion of E4ORF1+ HUVEC cells. The dose of E4ORF1+ endothelial cells infused in these subjects ranged from approximately 1x10⁶/kg to approximately 10x10⁶/kg. A third cohort in which subjects receive endothelial cells at a total dose of approximately 1x10⁹ cells (from approximately 10x10⁶/kg to approximately 20x10⁶/kg) is being initiated.

Thus, these clinical studies differ from those described in the previous Examples with respect to the patient population (leukemia or myelodysplastic syndrome patients as opposed to lymphoma patients), the cell transplant (allogeneic cord blood stem cells as opposed to autologous hematopoietic cells), the administration regimen (co-administration of the stem cell transplant with the E4ORF1+ HUVEC cells as opposed to initial administration of the stem cell transplant followed by a subsequent administration of the E4ORF1+ HUVEC cells), and the range of doses of E4ORF1+ HUVEC cells administered.

Adverse events in this study were coded using version 4.03 of the CTCAE. Of eight patients only two reported gastrointestinal adverse events that were deemed severe (Grade ≥ 3). One patient reported Grade 3 vomiting, as well as Grade 3 gastroparesis (a disorder of impaired gastric emptying), and Grade 3 typhlitis (inflammation of the caecum). The only other Grade 3 gastrointestinal (GI) toxicity reported was an event of food poisoning that was deemed unrelated to cytotoxic chemotherapy. The one patient with multiple GI adverse events (AEs) also reported several Grade 3 or higher events in other organ systems (heart, respiratory, CNS [encephalopathy], and vascular) culminating in respiratory failure and death from cardiac arrest. No cardiac adverse events of any Grade were reported in any other patient. Vascular AEs of Grade 3 or higher were reported in only one other patient (Grade 3 hypertension). Three patients reported Grade 3 febrile neutropenia. These patients all received endothelial cell doses of less than 20 x10⁶ endothelial cells per kg. Only one patient reported a hepatobiliary adverse event of Grade 3 or higher (Grade 3 hyperbilirubinemia); this event was considered associated with underlying acute graft-versus-host disease (GVHD) affecting the liver rather than to chemotherapy. One other patient reported Grade 3 acute GVHD. With respect to other gastrointestinal side effects: only two patients reported oral mucositis; both were deemed of Grade 2 (moderate). This incidence is lower than reported in the literature (19/20 patients reported mucositis in double cord blood transplant (Biol Blood Marrow Transplant. 2014 Dec;20(12):2062-6); Bone Marrow Transplant 2014 Jul;49(7):955-60.) Seven patients reported at least one event of nausea, vomiting, or diarrhea. This included the one patient reporting Grade 3 vomiting event listed above. All other N/V/D events were Grade 1 or Grade 2.

Other than as listed above only one subject reported a Grade 2 CNS AE (light headedness); three other patients reported Grade 1 CNS events AEs. Five patients reported psychiatric AEs; none more than Grade 2. Only two patients reported renal or urinary adverse events (one event each); both were deemed Grade 1.

Only two AEs were reported in the reproductive system or breast; one patient with Grade 2 vaginal bleeding and one with Grade 1 vaginal pruritis.

Other than as listed above, only four adverse events were reported in the respiratory, thoracic and mediastinal category- none higher than Grade 2. These included cough, hiccups, sore throat, and a nosebleed.

Only two cutaneous adverse events of Grade 2 were reported (one rash and one pruritis); six Grade 1 cutaneous AEs were reported.

Further, both the time to platelet engraftment and the time to neutrophil engraftment were positively associated with the infused dose of E4ORF1+ endothelial cells (Table 16 & **Fig. 11A-B**).

**Table 16 - Time to Platelet Engraftment & Neutrophil Engraftment**

| **Subject** | **E4ORF1+ Endothelial Cell Dose infused (10⁶/kg)** | **Time to Neutrophil Engraftment (days)** | **Time to Platelet Engraftment (days)** |
|---|---|---|---|
| **1** | 1.47 | 29 | Not evaluated |
| **2** | 2.74 | 35 | 50 |
| **3** | 2.82 | 21 | 35 |
| **4** | 3.23 | 19 | 42 |
| **5** | 4.48 | 22 | 35 |
| **6** | 6.77 | 16 | 37 |
| **7** | 7.13 | 21 | 28 |
| **8** | 8.45 | 12 | 22 |

### Reference List

Anderson, K.O., et al., Symptom burden in patients undergoing autologous stem-cell transplantation. Bone Marrow Transplant, 2007. 39(12): p. 759-66.
Bacigalupo et al., Defining the intensity of conditioning regimens working definitions, Biol Blood Marrow Transplant. 2009 December ; 15(12): 1628-1633.
Andrea Caballero, M.D., et al., BEAM chemotherapy followed by autologous stem cell support in lymphoma patients: analysis of efficacy, toxicity and prognostic factors. Bone Marrow Transplant, 1997. 20(6): p. 451-8.
Chaudhry et al., The Incidence and Severity of Oral Mucositis among Allogeneic Hematopoietic Stem Cell Transplantation Patients: A Systematic Review, Biol Blood Marrow Transplant 22 (2016) 605e616.
Eapen et al., "Mismatched Related and Unrelated Donors for Allogeneic Hematopoietic Cell Transplantation for Adults with Hematologic Malignancies, Biol Blood Marrow Transplant 20 (2014) 1485e1492.
Hafsa M. CChen, Y.B., et al., Impact of conditioning regimen on outcomes for patients with lymphoma undergoing high-dose therapy with autologous hematopoietic cell transplantation. Biol Blood Marrow Transplant, 2015. 21(6): p. 1046-1053.
Crump, M., et al., Outcomes in refractory diffuse large B-cell lymphoma: results from the international SCHOLAR-1 study. Blood, 2017. 130(16): p. 1800-1808.
Dahi, P.B., et al., Favorable outcomes in elderly patients undergoing high-dose therapy and autologous stem cell transplantation for non-Hodgkin lymphoma. Biol Blood Marrow Transplant, 2014. 20(12): p. 2004-9.
Dahi, P.B., et al., Toxicities associated with high dose chemotherapy and autologous stem cell transplantation in older patients with Non-Hodgkin lymphoma. Biol Blood Marrow Transplant, 2018. 24(3): p. S130.
Dahi, P.B., et al., Strategies to improve outcomes of autologous hematopoietic cell transplant in lymphoma. Bone Marrow Transplant, 2019. 54(7): p. 943-960.
de Vos, F.Y., et al., Long-term survivors of ovarian malignancies after cisplatin-based chemotherapy; cardiovascular risk factors and signs of vascular damage. Eur J Cancer, 2004. 40(5): p. 696-700.
Gisselbrecht, C., et al., Salvage regimens with autologous transplantation for relapsed large B-cell lymphoma in the rituximab era. J Clin Oncol, 2010. 28(27): p. 4184-90.
Gyurkocza, B. and B.M. Sandmaier, Conditioning regimens for hematopoietic cell transplantation: one size does not fit all. Blood, 2014. 124(3): p. 344-53.
Friedberg, J.W., Relapsed/refractory diffuse large B-cell lymphoma. Hematology Am Soc Hematol Educ Program, 2011. 2011: p. 498-505.
Jantunen, E., et al., Early treatment-related mortality in adult autologous stem cell transplant recipients: a nation-wide survey of 1482 transplanted patients. Eur J Haematol, 2006. 76(3): p. 245-50.
Loke et al., Outcomes of EAM conditioned autologous haematopoietic SCT for lymphoma. A matched pairs retrospective single-centre study analysis, Bone Marrow Transplantation (2013) 48, 1486-1487.
Majhail, N.S., et al., Indications for Autologous and Allogeneic Hematopoietic Cell Transplantation: Guidelines from the American Society for Blood and Marrow Transplantation. Biol Blood Marrow Transplant, 2015. 21(11): p. 1863-1869.
Mills, W., et al., BEAM chemotherapy and autologous bone marrow transplantation for patients with relapsed or refractory non-Hodgkin's lymphoma. J Clin Oncol, 1995. 13(3): p. 588-95.
National_Institute_for_Health_and_Care_Excellence, Polatuzumab vedotin with rituximab and bendamustine for treating relapsed or refractory diffuse large B cell lymphoma [ID1576], N.I.f.H.a.C. Excellence, Editor. 2019.
Nuver, J., et al., Vascular damage in testicular cancer patients: a study on endothelial activation by bleomycin and cisplatin in vitro. Oncol Rep, 2010. 23(1): p. 247-53.
Olivieri, J., et al., A Comparison of the Conditioning Regimens BEAM and FEAM for Autologous Hematopoietic Stem Cell Transplantation in Lymphoma: An Observational Study on 1038 Patients From Fondazione Italiana Linfomi. Biol Blood Marrow Transplant, 2018. 24(9): p. 1814-1822.
Pamukcuoglu et al., "Hematopoietic Cell Transplant-Related Toxicities and Mortality in Frail Recipients," Biol. Blood Marrow Transplant, 2019; 25(12): 2454-2460.
Perales et al., Real-World Economic Burden Associated with Transplantation-Related Complications, Biol Blood Marrow Transplant 23 (2017) 1788-1794.
Philip, T., et al., Autologous bone marrow transplantation as compared with salvage chemotherapy in relapses of chemotherapy-sensitive non-Hodgkin's lymphoma. N Engl J Med, 1995. 333(23): p. 1540-5.
Philip, T., et al., High-dose therapy and autologous bone marrow transplantation after failure of conventional chemotherapy in adults with intermediate-grade or high-grade non-Hodgkin's lymphoma. N Engl J Med, 1987. 316(24): p. 1493-8.
Scordo et al, 2017, "A Comprehensive Assessment of Toxicities in Patients with Central Nervous System Lymphoma Undergoing Autologous Stem Cell Transplantation Using Thiotepa, Busulfan, and Cyclophosphamide Conditioning" Biol. Blood Marrow Transplant 23 (2017) 38-43.
Sellner, L., et al., Thiotepa-based high-dose therapy for autologous stem cell transplantation in lymphoma: a retrospective study from the EBMT. Bone Marrow Transplant, 2016. 51(2): p. 212-8.
Stansborough, R.L., et al., Radiotherapy-induced gut toxicity: Involvement of matrix metalloproteinases and the intestinal microvasculature. Int J Radiat Biol, 2016. 92(5): p. 241-8.
Stiff, P., Mucositis associated with stem cell transplantation: current status and innovative approaches to management. Bone Marrow Transplant, 2001. 27 Suppl 2: p. S3-S11.
Taplitz, R.A., et al., Antimicrobial Prophylaxis for Adult Patients With Cancer-Related Immunosuppression: ASCO and IDSA Clinical Practice Guideline Update. J Clin Oncol, 2018. 36(30): p. 3043-3054.
von Tempelhoff, G.F., et al., Blood coagulation during adjuvant epirubicin/cyclophosphamide chemotherapy in patients with primary operable breast cancer. J Clin Oncol, 1996. 14(9): p. 2560-8.
Vose, J.M., et al., Phase I trial of iodine-131 tositumomab with high-dose chemotherapy and autologous stem-cell transplantation for relapsed non-Hodgkin's lymphoma. J Clin Oncol, 2005. 23(3): p. 461-7.
Loke et al., Outcomes of EAM conditioned autologous haematopoietic SCT for lymphoma. A matched pairs retrospective single-centre study analysis, Bone Marrow Transplantation (2013) 48, 1486-1487.

The invention may additionally be understood with reference to the following numbered paragraphs:
Paragraph 1. A method of mitigating a non-hematologic severe regimen-related toxicity (SRRT) in a human subject in need thereof, the method comprising intravenously administering a therapeutic composition comprising an effective amount of E4ORF1+ HUVECs to an adult human subject with Hodgkin's lymphoma or non-Hodgkin's lymphoma, wherein: (a) the effective amount is from about 10x106 cells per kg bodyweight to about 30x106 cells per kg bodyweight, (b) the subject has previously received high-dose therapy (HDT) and an autologous hematopoietic stem cell transplant (AHCT), (c) the E4ORF1+ HUVECs are administered to the subject on the same day that the subject receives the AHCT, and (d) the non-hematologic SRRT is selected from the group consisting of an oral / gastrointestinal SSRT and febrile neutropenia, thereby mitigating the SSRT in the subject.
Paragraph 2. The method of paragraph 1, wherein the E4ORF1+ HUVECs are administered to the subject from about 2 hours to about 4 hours after the AHCT has been completed.
Paragraph 3. The method of paragraph 1, wherein the effective amount is from about 10x10⁶ cells per kg to about 20x10⁶ cells per kg bodyweight.
Paragraph 4. The method of paragraph 1, wherein the effective amount is about 20x10⁶ cells per kg bodyweight.
Paragraph 5. The method of paragraph 1, further comprising subsequently administering a second dose of the therapeutic composition to the subject.
Paragraph 6. The method of paragraph 5, wherein the second dose is administered from about 1 day to about 3 days after the AHCT is completed.
Paragraph 7. The method of paragraph 1, wherein the subject is at least 40 years old.
Paragraph 8. The method of paragraph 1, wherein the subject is at least 60 years old.
Paragraph 9. The method of paragraph 1, wherein the therapeutic composition comprises E4ORF1+ HUVECs in a physiological saline.
Paragraph 10. The method of paragraph 1, wherein the therapeutic composition further comprises one or more excipients selected from the group consisting of buffers, salts, polysaccharides, proteins and preservatives.
Paragraph 11. The method of paragraph 1, wherein the therapeutic composition further comprises human serum albumin (HSA).
Paragraph 12. The method of paragraph 1, wherein the therapeutic composition further comprises Dextran40.
Paragraph 13. The method of paragraph 1, wherein the therapeutic composition further comprises about 0.25% DMSO.
Paragraph 14. The method of paragraph 1, wherein the therapeutic composition comprises about 5x10⁶ E4ORF1+ HUVECs per ml.
Paragraph 15. The method of paragraph 1, wherein the oral / GI SSRT is selected from the group consisting of mucositis, stomatitis, typhlitis, nausea, vomiting, and diarrhea.
Paragraph 16. The method of paragraph 15, wherein the mucositis is selected from oral, esophageal, gastric, small intestinal, large intestinal, colonic, rectal or anal mucositis.
Paragraph 17. The method of paragraph 1, wherein the AHCT comprises transplantation of bone marrow hematopoietic stem cells.
Paragraph 18. The method of paragraph 1, wherein the AHCT comprises transplantation of peripheral blood hematopoietic stem cells.
Paragraph 19. The method of paragraph 1, wherein the high dose therapy (HDT) comprises administration to the subject of a combination of chemotherapeutic agents selected from the group consisting of BEAM, BeEAM, FEAM, CBV, TBC, DHAP and Bu/Cy/Flu.
Paragraph 20. The method of paragraph 1, wherein the method also results in the mitigation of a hematologic SRRT.
Paragraph 21. The method of paragraph 20, wherein the hematologic SRRT comprises thrombocytopenia, neutropenia, leukopenia, or anemia.
Paragraph 22. The method of paragraph 20, wherein the time to neutrophil engraftment or platelet engraftment is reduced as compared to the time to neutrophil engraftment or platelet engraftment in a subject not treated with the therapeutic composition.
Paragraph 23. A method of mitigating a non-hematologic severe regimen-related toxicity (SRRT) in a human subject in need thereof, the method comprising:
   administering a therapeutic composition comprising an effective amount of endothelial cells (ECs) to a human subject who has undergone, is undergoing, or will undergo chemotherapy, wherein the method results in mitigation of a SRRT in the subject.
Paragraph 24. The method of paragraph 23, wherein the SRRT is selected from the group consisting of an oral/GI toxicity, febrile neutropenia, a solid organ toxicity, veno-occlusive disease, and a severe infection.
Paragraph 25. The method of paragraph 23, wherein the ECs are human umbilical vein endothelial cells (HUVECs).
Paragraph 26. The method of paragraph 23, wherein the ECs are E4ORF1+ human umbilical vein endothelial cells (E4ORF1+ HUVECs).
Paragraph 27. The method of paragraph 23, wherein the subject has a malignant disease.
Paragraph 28. The method of paragraph 27, wherein the malignant disease is a lymphoma.
Paragraph 29. The method of paragraph 27, wherein the malignant disease is leukemia.
Paragraph 30. The method of paragraph 27, wherein the malignant disease is multiple myeloma.
Paragraph 31. The method of paragraph 27, wherein the malignant disease is myelodysplastic syndrome (MDS).
Paragraph 32. The method of paragraph 28, wherein the subject has a lymphoma selected from the group consisting of: Hodgkins's lymphoma (HL) and non-Hodgkins's lymphoma (NHL).
Paragraph 33. The method of paragraph 28, wherein the subject has lymphoma without CNS involvement.
Paragraph 34. The method of paragraph 28, wherein the subject has lymphoma with CNS involvement (CNS lymphoma).
Paragraph 35. The method of paragraph 28, wherein the subject has relapsed-refractory lymphoma.
Paragraph 36. The method of paragraph 23, wherein the chemotherapy is high dose therapy (HDT).
Paragraph 37. The method of paragraph 23, wherein the chemotherapy is myeloablative chemotherapy.
Paragraph 38. The method of paragraph 23, wherein the chemotherapy comprises administration to the subject of a combination of chemotherapeutic agents selected from the group consisting of BEAM, BeEAM, FEAM, CBV, TBC, DHAP and Bu/Cy/Flu.
Paragraph 39. The method of paragraph 23, wherein the chemotherapy is administered to the subject as part of a conditioning regimen to prepare the subject for receipt of a hematopoietic stem cell transplant.
Paragraph 40. The method of paragraph 23, wherein the subject has received a hematopoietic stem cell transplant.
Paragraph 41. The method of paragraph 23, wherein the subject has received a hematopoietic stem cell transplant at the time that the therapeutic composition is administered to the subject.
Paragraph 42. The method of paragraph 23, wherein the subject has received a hematopoietic stem cell transplant within about 3 days of the time that the therapeutic composition is administered to the subject.
Paragraph 43. The method of paragraph 23, wherein the subject receives a hematopoietic stem cell transplant on the same day that the therapeutic composition is administered to the subject.
Paragraph 44. The method of paragraph 23, wherein the subject receives a hematopoietic stem cell transplant within about 1 to about 4 hours of the time that the therapeutic composition is administered to the subject.
Paragraph 45. The method of paragraph 23, wherein the subject receives a hematopoietic stem cell transplant at the same time that the therapeutic composition is administered to the subject.
Paragraph 46. The method of paragraph 45, wherein the hematopoietic stem cell transplant and the therapeutic composition are administered to the subject in the same IV infusion.
Paragraph 47. The method of any of paragraphs 39-46, wherein the hematopoietic stem cell transplant is an autologous hematopoietic stem cell transplant (ASCT).
Paragraph 48. The method of any of paragraphs 39-46, wherein the hematopoietic stem cell transplant is an allogeneic hematopoietic stem cell transplant (AlloSCT).
Paragraph 49. The method of any of paragraphs 39-48, wherein the subject receives high-dose therapy (HDT) prior to the hematopoietic stem cell transplant.
Paragraph 50. The method of any of paragraphs 23-49, wherein the therapeutic composition comprises ECs at a concentration of about 5 million cells per ml.
Paragraph 51. The method of any of paragraphs 23-50, wherein the therapeutic composition comprises endothelial cells in a physiological saline.
Paragraph 52. The method of any of paragraphs 23-51, wherein the therapeutic composition comprises one or more excipients selected from the group consisting of buffers, salts, polysaccharides, proteins and preservatives.
Paragraph 53. The method of any of paragraphs 23-52, wherein the therapeutic composition comprises human serum albumin (HSA).
Paragraph 54. The method of any of paragraphs 23-53, wherein the therapeutic composition comprises Dextran40.
Paragraph 55. The method of any of paragraphs 23-50, wherein the therapeutic composition comprises DMSO.
Paragraph 56. The method of any of paragraphs 23-55, wherein the therapeutic composition is administered to the subjects intravenously (IV).
Paragraph 57. The method of any of paragraphs 23-56, wherein the therapeutic composition is administered to the subject in single IV infusion.
Paragraph 58. The method of any of paragraphs 23-56, wherein the therapeutic composition is administered to the subject in multiple IV infusions.
Paragraph 59. The method of paragraph 58, wherein the therapeutic composition is administered to the subject in a first IV infusion on Day 0 and a second IV infusion on Day 1, Day 2, Day 3, Day 4, or Day 5.
Paragraph 60. The method of paragraph 58, wherein the therapeutic composition is administered to the subject in a first IV infusion on Day 0 and a second IV infusion on Day 3.
Paragraph 61. The method of paragraph 58, wherein the therapeutic composition is administered to the subject in a first IV infusion on Day 0 and a second IV infusion on Day 2.
Paragraph 62. The method of paragraph 58, wherein the therapeutic composition is administered to the subject in a first IV infusion on Day 0 and a second IV infusion on Day 1.
Paragraph 63. The method of any of paragraphs 23-62, wherein the effective amount is about 5x10⁶ cells per kg bodyweight.
Paragraph 64. The method of paragraphs 23-62, wherein the effective amount is about 10x10⁶ cells per kg bodyweight.
Paragraph 65. The method of any of paragraphs 23-62, wherein the effective amount is about 15x10⁶ cells per kg bodyweight.
Paragraph 66. The method of any of paragraphs 23-62 wherein the effective amount is about 20x10⁶ cells per kg bodyweight.
Paragraph 67. The method of any of paragraphs 23-62, wherein the effective amount is about 25x10⁶ cells per kg bodyweight.
Paragraph 68. The method of any of paragraphs 23-62, wherein the effective amount is about 30x10⁶ cells per kg bodyweight.
Paragraph 69. The method of any of paragraphs 23-62, wherein the effective amount is about 35x10⁶ cells per kg bodyweight.
Paragraph 70. The method of any of paragraphs 23-62, wherein the effective amount is about 40x10⁶ cells per kg bodyweight.
Paragraph 71. The method of any of paragraphs 23-62, wherein the effective amount is from about 5x10⁶ cells per kg bodyweight to about 40x10⁶ cells per kg bodyweight.
Paragraph 72. The method of any of paragraphs 23-62, wherein the effective amount is from about 5x10⁶ cells per kg bodyweight to about 25x10⁶ cells per kg bodyweight.
Paragraph 73. The method of any of paragraphs 23-62 wherein the effective amount is from about 10x10⁶ cells per kg bodyweight to about 20x10⁶ cells per kg bodyweight.
Paragraph 74. The method of any of paragraphs 23-62 wherein the effective amount is about 20x10⁶ cells per kg bodyweight.
Paragraph 75. The method of any of paragraphs 23-74, wherein the non-hematologic SRRT that is mitigated is selected from the group consisting of mucositis oral, esophageal, gastric, small intestinal, large intestinal, colonic, rectal or anal mucositis), stomatitis, typhlitis, nausea, vomiting, and diarrhea.
Paragraph 76. The method of any of paragraphs 23-75, wherein the method also results in the mitigation of a hematologic SRRT.
Paragraph 77. The method of paragraph 76, wherein the hematologic SRRT comprises thrombocytopenia, neutropenia, leukopenia, or anemia.
Paragraph 78. The method of paragraph 76, wherein the time to neutrophil engraftment or platelet engraftment is reduced as compared to the time to neutrophil engraftment or platelet engraftment in a subject not treated with the therapeutic composition.
Paragraph 79. The method of any of paragraphs 23-79, wherein the ECs are EAORF1+ ECs.
Paragraph 80. The method of any of paragraphs 23-78, wherein the ECs are E4ORF1+ HUVECs
Paragraph 81. A method of mitigating a chemotherapy side effect in a human subject in need thereof, the method comprising: administering a therapeutic composition comprising an effective amount of endothelial cells (ECs) to a human subject who has undergone, is undergoing, or will undergo chemotherapy, wherein the method results in mitigation of the chemotherapy side effect in the subject.
Paragraph 82. The method of paragraph 81, wherein the ECs are E4ORF1+ ECs.
Paragraph 83. The method of paragraph 81, wherein the ECs are E4ORF1+ HUVECs.

## Claims

1. A therapeutic composition comprising E4ORF1+ human umbilical vein endothelial cells (HUVECs) in solution suitable for administration to a human subject.

2. The therapeutic composition of claim 1, wherein the E4ORF1+ HUVECs is at a concentration of about 5x10⁶ E4ORF1+ HUVECs per ml.

3. The therapeutic composition of claims 1 or 2, wherein the therapeutic composition further comprises one or more additional components selected from the group consisting of buffers, salts, polysaccharides, proteins and preservatives.

4. The therapeutic composition of claim 3, wherein the therapeutic composition further comprises human serum albumin (HSA), optionally at a concentration of about 4.5 mg/ml.

5. The therapeutic composition of claim 3, wherein the therapeutic composition further comprises Dextran40, optionally at a concentration of about 8 mg/ml.

6. The therapeutic composition of claim 3, wherein the therapeutic composition further comprises DMSO, optionally at about 0.25% DMSO.

7. A therapeutic composition according to any one of the preceding claims, for use in intravenous administration to a human subject in need thereof.

8. A therapeutic composition for use according to claim 7, wherein
from about 5x10⁶ E4ORF1+ HUVECs per kg bodyweight to about 40x10⁶ E4ORF1+ HUVECs per kg bodyweight are administered to the subject;
optionally wherein from about 5x10⁶ E4ORF1+ HUVECs per kg bodyweight to about 25x10⁶ E4ORF1+ HUVECs per kg bodyweight are administered to the subject;
further optionally wherein from about 10x10⁶ E4ORF1+ HUVECs per kg bodyweight to about 20x10⁶ E4ORF1+ HUVECs per kg bodyweight are administered to the subject.

9. A therapeutic composition for use according to claims 7 or 8, wherein the therapeutic composition is administered to the subjects once or twice or three or more times.

10. A therapeutic composition for use according to any one of claims 7-9, wherein the therapeutic composition is administered to the subjects twice, with the first dose administered on day 0 and the second dose administered on day 1, 2, 3, 4, 5, 6 or 7.

11. A therapeutic composition for use according to any one of claims 7-10, wherein the therapeutic composition is administered to the subjects by IV infusion over a period of about 1 hour and/or at a rate of about 6ml/minute or of about 30x10⁶ E4ORF1+ HUVECs/minute.

12. A therapeutic composition for use according to any one of claims 7-11, wherein the therapeutic composition is for use in treating subjects having cancer/malignancy,
optionally wherein the cancer is a hematologic cancer/malignancy and/or non-hematologic cancer/malignancy,
further optionally wherein the hematologic cancer/malignancy is leukemia, lymphoma, and/or myelodysplastic syndrome.

13. A therapeutic composition for use according to claim 12, wherein
(a) the leukemia is acute myeloid leukemia (AML) and/or acute lymphoblastic leukemia (ALL); and/or
(b) the lymphoma is Hodgkins's lymphoma (HL), non-Hodgkins's lymphoma (NHL), large B cell lymphoma (DLBCL), T cell lymphoma (TCL), a lymphoma without CNS involvement, a lymphoma with CNS involvement (i.e., "CNS lymphoma"), relapsed lymphoma, and/or refractory lymphoma, optionally wherein the NHL is multiple myeloma.

14. A therapeutic composition for use according to claim 12, wherein the non-hematologic cancer/malignancy is selected from the group consisting of an adrenocortical tumor, alveolar soft part sarcoma, astrocytoma, breast cancer, bladder cancer, brain cancer, carcinoma, cervical cancer, chondrosarcoma, colorectal cancer, desmoid tumors, desmoplastic small round cell cancer, endocrine cancer, endodermal sinus tumor, endometrial cancer, epithelioid hemangioendothelioma, esophageal cancer, Ewing sarcoma, gastric cancer, germ cell tumor, glioma, heart cancer, hepatoblastoma, hepatocellular carcinoma, lip and/or oral cancer, lung cancer, melanoma, mesothelioma, nephroma, neuroblastoma, non-rhabdomyosarcoma soft tissue sarcoma, oropharangeal cancer, osteosarcoma, ovarian cancer, pancreatic cancer, paraspinal sarcoma, prostate cancer, rectal cancer, renal cell carcinoma, retinoblastoma, rhabdomyosarcoma, small cell lung cancer, synovial sarcoma, testicular cancer, throat cancer, thyroid cancer, urethral cancer, uterine sarcoma, and Wilms Tumor.

15. A kit comprising a therapeutic composition according to any one of claims 1-7 in sterile packaging and instructions for administering the therapeutic composition to a human subject.
